# NEW EUROPEAN PATENT SPECIFICATION

(11) **EP 0 601 092 B2**
(45) Date of publication and mention of the opposition decision: **15.11.2006**
(45) Mention of the grant of the patent: 07.07.1999
(21) Application number: 92919292.0
(22) Date of filing: 27.08.1992
(51) Int. Cl.: C12N 15/65, C12N 15/56, C12N 15/82, C12N 5/04, C07H 17/02, C07H 15/203, A01H 5/00

(54) **METHOD FOR THE SELECTION OF GENETICALLY TRANSFORMED CELLS AND COMPOUNDS FOR USE IN THE METHOD**
VERFAHREN ZUR SELEKTION GENETISCH TRANSFORMIERTER ZELLEN UND DAFÜR VERWENDBARE VERBINDUNGEN
PROCEDE DE SELECTION DE CELLULES TRANSFORMEES GENETIQUEMENT ET COMPOSES DESTINES A ETRE UTILISES DANS LEDIT PROCEDE

(30) Priority: 28.08.1991 DK 152291
(43) Date of publication of application: 15.06.1994
(60) Divisional application: 98121031.3
(73) Proprietor: Syngenta Participations AG, 4058 Basel (CH)
(72) Inventor: OKKELS, Finn, T., DK-4000 Roskilde (DK); WHENHAM, Robert, J., Leicester LE9, 8JA (GB)
(74) Representative: Sheard, Andrew Gregory
(86) International application number: PCT/DK1992/000252
(87) International publication number: WO 1993/005163

(56) References cited:
- WO-A-89/03880
- US-A- 4 857 467
- NATURE, vol. 342, 14 December 1989, London (GB); R.A. JEFFERSON et al., pp. 837-838
- CHEMICAL ABSTRACTS, vol. 52, 1958, Columbus, OH (US); J.A.R. MEAD et al., AN 7468h
- GENE, vol. 101, no. 2, 30 May 1991, Amsterdam (NL)M; R.S.S. DATLA et al., pp. 239-246

## Description

### FIELD OF THE INVENTION

The present invention relates to a method for selecting genetically transformed plant cells into which a desired nucleotide sequence has been incorporated by providing the transformed cells with a selective advantage without damaging the non-transformed cells, as well as to novel compounds for use in the method.

### BACKGROUND OF THE INVENTION

It is well known that when new genetic material is to be introduced into a population of cells by transformation, only a certain number of the cells are successfully transformed, i.e. receive the new genetic material. It is then necessary to identify the genetically transformed cells so that these cells may be separated from the non-transformed cells in the population. Identification and separation of the transformed cells have traditionally been accomplished using what may be termed "negative selection", in other words by use of a method whereby the transformed cells are able to survive and grow, while the non-transformed cells are subjected to growth inhibition or perhaps even killed by a substance which the transformed cells are able to tolerate.

For example, when a population of plant cells is subjected to genetic transformation, selection of the transformed cells typically takes place using a selection gene which codes for antibiotic or herbicide resistance. The selection gene - which in itself generally has no useful function in the genetically transformed plant, and may in fact be undesirable in the plant - is coupled to or co-introduced with the gene to be incorporated into the plant in question, so that both of the two genes are incorporated into the population of cells, or rather into certain of the cells in the population, since it is not possible in practice to transform all or even a majority of the cells. The cells are then cultivated on or in a medium containing the antibiotic or herbicide to which the genetically transformed cells are resistant by virtue of the selection gene, thereby allowing the transformed cells to be identified, since the non-transformed cells - which do not contain the antibiotic or herbicide resistance gene in question - are subjected to growth inhibition or are killed.

These negative selection methods have, however, certain disadvantages. First of all, the non-transformed cells may die because of the presence of e.g. antibiotics in the growth medium. As a result, when the population of cells is a coherent tissue there is a distinct risk that not only the non-transformed cells but also the transformed cells may die, due to the fact that the death of the non-transformed cells may cut off the supply of nutrients to the transformed cells or because the damaged or dying non-transformed cells may excrete toxic compounds.

Another significant disadvantage of negative selection is that the presence of an unnecessary gene for e.g. antibiotic resistance may be undesirable. For instance, there is concern among environmental groups and governmental authorities about whether it is safe to incorporate genes coding for antibiotic resistance into plants and microorganisms. This concern is of particular significance for food plants and for microorganisms which are not designed to be used in a closed environment (e.g. microorganisms for use in agriculture), as well as for microorganisms which are designed for use in a closed environment, but which may accidently be released from the closed environment. While these concerns may prove to be unfounded, such concerns may nevertheless lead to governmental restrictions on the use of antibiotic resistance genes in e.g. plants, and it is therefore desirable to develop new methods for selecting genetically transformed cells which are not dependent on such genes.

A further disadvantage of negative selection is that plant tissues or cells treated with toxic substances become more susceptible to bacterial infection. This is a problem when Agrobacterium is used as a transformation vector, because the treated tissues or cells sometimes become overgrown with the bacteria even though antibiotics are used to prevent bacteria growth.

In addition, selection of cells or tissues using negative selection requires very precise timing of expression of the introduced genes in relation to the selection process. If the transgenic cells are treated with a toxic compound before the detoxifying gene is expressed or before enough gene products are produced to antagonize the action of the toxic compound, the transgenic cells will be killed together with the non-transgenic cells. If selection is performed too late, the selection of transgenic cells or tissues may be hindered by e.g. shoot formation from non-transgenic cells or tissues.

The above disadvantages are eliminated by the method according to the present invention (termed "positive selection"), which for the first time makes it possible to identify and isolate genetically transformed plant cells without damaging or killing the non-transformed cells in the population and without co-introduction of antibiotic or herbicide resistance genes. In addition to the fact that the need for antibiotic or herbicide resistance genes is eliminated, it has been shown that the positive selection method according to the present invention is often far more efficient than traditional negative selection. As described below in the Examples, the number of transgenic shoots selected from tobacco leaf discs using positive selection is e.g. on the order of 30 times higher than the number of shoots selected using a traditional kanamycin-based negative selection system, and a combination of positive and negative selection gave a selection frequency of transgenic shoots of about 10 times that obtained using negative selection alone (see Example 11). Furthermore, the use of positive selection provides the advantage that a single gene may be used as both a reporter gene and a selection gene, resulting in simplification of vector constructions, more stable constructions and a 100% correlation between the expression of reporter and selection genes. Positive selection also eliminates the above-mentioned problems with regard to timing, since the compounds resulting in selection will always be produced as a consequence of gene expression. Thus, the selective compound will accumulate when the selection gene is expressed, the selection effect appearing when a sufficient amount of the selective compound has been produced.

Means of monitoring gene activity within transformed cells have been known previously in the prior art.

Jefferson RA (1989) Nature Vol 342, 837-838 disclose the use of GUS and the glucuronide permease system as a monitoring system for transformed plant cells. Jefferson specifically refers to the hydrolysis of the X-Gluc substrate and the detection of the blue Indigo dye product In transformed plant cells containing the 35S promoter-gus A gene construct.

US-P 4,857,467 discloses transformation of yeast strains of the genus *Pichia* with DNA fragments encoding gene functions which the host lacks or in which the host is deficient. The transformed yeast cells are capable of growth on a carbon and energy source which requires expression of the gene function provided by the DNA fragment.

WO 89/03880 discloses transfection of host cells with a gene encoding glucuronide permease. The system may be used together with GUS gene fusions.

None of the cited art documents disclose a method for selecting transformed plant cells based on the presence of a particular introduced nucleotide sequence as taught within the scope of the present invention.

### BRIEF DISCLOSURE OF THE INVENTION

One aspect of the present invention relates to a method for selecting genetically transformed plant cells from a population of cells, wherein the genetically transformed plant cells are transformed with a desired expressible nucleotide sequence containing a regulatory sequence enabling its expression in the transformed cells and a co-introduced expressible nucleotide sequence also containing a regulatory sequence enabling its expression in the transformed cells, which method comprises supplying the said population with a compound which can be metabolized by the expression product of the co-introduced expressible nucleotide sequence which has been introduced with the desired expressible nucleotide sequence into the said transformed cells, so as to provide the transformed cells with a physiological advantage when compared to the non-transformed cells, wherein the compound is not an antibiotic or herbicide and has no direct adverse effect on the non-transformed cells.

In one embodiment, the method is performed by cultivating the population of cells on or in a medium containing at least one inactive compound or nutrient which is directly or indirectly activated in cells containing the co-introduced nucleotide sequence and the desired nucleotide sequence, the compound or nutrient being inactive in non-transformed cells or less active in non-transformed cells than in transformed cells, such that the transformed cells are provided with a selective advantage allowing them to be selected from the cell population.

In another embodiment, the method is performed by cultivating the population of cells on or in a medium containing at least one compound or nutrient which is made available for the transformed cells by expression or transcription of the co-introduced nucleotide sequence, the compound or nutrient not being available for non-transformed cells or being less available for non-transformed cells than for transformed cells, such that the transformed cells are provided with a selective advantage allowing them to be selected from the cell population.

In a further embodiment, expression of the co-introduced nucleotide sequence leads to an increase in the activity of an enzyme found endogenously in the population of cells, such that the activity of the enzyme in transformed cells is greater than the activity of the enzyme in non-transformed cells.

Compounds which are suitable for use in the above method are those of the general formula I 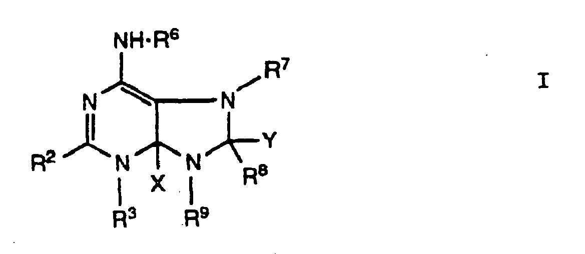 wherein
- R²: is H, CH₃, S-CH₃, SO₂-CH₃, SCH₂-phenyl, SH, OH, Cl or a group -S-R¹⁰, -NH-R¹⁰ or -O-R¹⁰, where R¹⁰ is a β-D-glucopyranuronosyl group [the structure of which is apparent from the working examples herein] or a salt thereof or an ester or amide derivative thereof at the carboxylic acid function,
- R⁶: is benzyl which may be substituted on the phenyl ring with OH, C₁₋₆-alkoxy, halogen, C₁₋₄-alkyl, NH₂ or CF₃, or with -O-R¹⁰, -S-R¹⁰ or -NH-R¹⁰, where R¹⁰ is as defined above; C₁₋₈-alkyl or C₂₋₈-alkenyl which may be substituted with from 1 to 3 hydroxy, glucosyloxy or C₁₋₆-alkoxy groups, with phenyl, and/or with -O-R¹⁰, -S-R¹⁰ or -NH-R¹⁰, where R¹⁰ is as defined above; esterified C₁₋₆-alkyl or C₂₋₆-alkenyl; furfuryl; or cyclohexylureido, phenylureido or tolylureido;
either
i) R⁷ and Y are half-bonds which together form a bond,
ii) one of R³ and R⁹ is H or a group R¹⁰ as defined above and the other is a half-bond which together with a half-bond X forms a bond, or R⁹ is ribosyl, 5'-phosphoribosyl, glucosyl or -CH₂CH(NH₂)COOH and R³ is a half-bond which together with the half-bond X forms a bond, and
iii) R⁸ is H, CH₃, S-CH₃, SO₂-CH₃, SCH₂-phenyl, SH, OH, Cl or a group -S-R¹⁰, -NH-R¹⁰ or -O-R¹⁰, where R¹⁰ is as defined above,
   or
iv) R⁷ is ribosyl, 5'-phosphoribosyl or glucosyl, R⁸ is H, R⁹ and Y are half-bonds which together form a bond, and R³ is a half-bond which together with the half-bond X forms a bond;
with the proviso that one of R², R³, R⁶, R⁸ and R⁹ is or comprises a β-D-glucopyranuronosyl group, or a salt thereof or an ester or amide derivative thereof at the carboxylic acid function.

Additional compounds which may be used in the above method are those of the general formula II 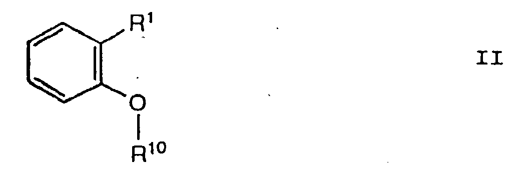 wherein
- R¹: is cis- -CH=CH-COOH, a salt thereof or an ester derivative thereof at the carboxylic acid function, or the amide derivative of cis- and/or trans- -CH=CH-COOH, and
- R¹⁰: is as defined above.

In addition, disclosed herein are genetically transformed cells which have been selected according to the above method, in particular plant cells, as well as plants, progeny and seeds derived from such genetically transformed plant cells.

### DETAILED DISCLOSURE OF THE INVENTION

The term "cells" in the context of the present invention is intended to refer to any type of cells from which individual genetically transformed cells may be identified and isolated using the method of the invention, including plant cells, animal cells and microorganisms such as bacteria, fungi, yeast, etc. Furthermore, the term "cells" is also meant to encompass protoplasts, i.e. the protoplasm of a cell enclosed in a membrane but without a cell wall. While it is contemplated that the general principle of the present invention may be employed on any type of cells, the method has been found to be particularly suitable for the selection of genetically transformed plant cells.

The term "population of cells" refers to any group of cells which has been subjected to genetic transformation and from which it is desired to identify those cells which have been genetically transformed and to isolate the genetically transformed cells from non-genetically transformed cells. The population may e.g. be a tissue, an organ or a portion thereof, a population of individual cells in or on a substrate, e.g. a population of cells in a solution or suspension, or a whole organism, e.g. an entire plant.

The term "selecting" refers to the process of identifying and/or isolating the genetically transformed cells from the non-genetically transformed cells using the method disclosed herein.

The "desired nucleotide sequence" may be any nucleotide sequence which is to be incorporated into the cells in question to produce genetically transformed cells. Introduction of nucleotide sequences into plants, microorganisms and animals is widely practiced, and there are no limitations upon the nucleotide sequences whose presence may be detected by use of the positive selection method described herein. By use of this method the presence of the desired nucleotide sequence in the genetically transformed cells may be determined without the above-mentioned disadvantages associated with traditional negative selection systems.

The fact that a nucleotide sequence is "co-introduced with" the desired nucleotide sequence refers to the fact that the two nucleotide sequences are coupled to each other or otherwise introduced together in such a manner that the presence of the co-introduced nucleotide sequence in a cell indicates that the desired nucleotide sequence has been introduced into the cell, i.e. if one of the nucleotide sequences is shown to have been introduced, the probability that the other one also has been introduced is significantly increased. The two nucleotide sequences are thus typically, although not necessarily, part of the same genetic construct and are e.g. introduced by the same vector.

The methods described herein may also be used when the co-introduced nucleotide sequence and the desired nucleotide sequence are introduced independently. This may e.g. be performed by using the same bacteria for incorporation of both genes and incorporating a relatively large number of copies of the desired nucleotide sequence into the cells, whereby the probability is relatively high that cells which are shown to express the co-introduced nucleotide sequence also will contain and express the desired nucleotide sequence. Independent introduction of two or more genes resulting in co-expression of the genes in the same cell is generally expected to have a low probability, and the improved selection frequencies obtained by the positive selection method are therefore expected be especially advantageous in such systems.

Since it is necessary that the introduced nucleotide sequences are expressed in the transformed cells, a genetic construct containing the two nucleotide sequences will typically, but not necessarily, contain regulatory sequences enabling expression of the nucleotide sequences, e.g. known promotors and transcription terminators. Thus, the co-introduced nucleotide sequence will typically be associated with a promotor, which may be a constitutive or regulatable promotor, and the desired nucleotide sequence will typically also be associated with a constitutive or regulatable promotor.

As mentioned above, the method is particularly suitable for the selection of genetically transformed plant cells, thereby allowing identification and isolation of such cells without the use of selection genes coding for antibiotic or herbicide resistance. As with traditional negative selection methods, the positive selection method described herein may be used for selecting cells in vitro. However, the positive selection method may also be employed in vivo. Use of the positive selection method in vivo is of particular relevance e.g. in connection with genetic transformation performed on whole plants or on plant parts, in which the plants or plant parts comprise both transformed and non-transformed cells, since selection of the transformed cells is achieved without directly damaging the neighbouring non-transformed cells. The transformed cells thus have a selective "advantage" compared to the non-transformed cells (e.g. the ability to form shoots), but the non-transformed cells do not suffer any severe disadvantage in the sense of being damaged or killed, as is the case with negative selection using antibiotics or herbicides.

The "inactive compound or nutrient' may be any compound or nutrient in inactive or precursor form, i.e. which in the absence of expression of the co-introduced nucleotide sequence exists in a form which is substantially biologically inactive with respect to the cells in question, but which when the co-introduced nucleotide sequence is expressed or transcribed is hydrolyzed or otherwise activated or metabolized so as to provide the genetically transformed cells containing the desired nucleotide sequence with a selective advantage, thereby allowing them to be identified and isolated. The inactive compound or nutrient may thus e.g. be an inactive plant growth regulator, for example an inactivated cytokinin, auxin or gibberellin, a vitamin, e.g. inactivated thiamine, a carbohydrate (e.g. mannose, when the co-introduced nucleotide sequence encodes mannose-6-phosphate isomerase, or galactose or a galactose-containing compound, when the co-introduced nucleotide sequence encodes UDP-galactose-4-epimerase), a nitrogen-containing compound (e.g. an opine, when the co-introduced nucleotide sequence encodes an opine metabolism or transport enzyme), starch, a protein, or another nutrient in inactive form, or a compound which has an essential function during differentiation and dedifferentiation of cells and tissues. Treatment of cells and tissues with compounds inducing dependence on supplementary addition of essential compounds may also be used together with the corresponding inactive compounds. This approach may e.g. be used when sterol or saponin synthesis is inhibited and inactive sterols or saponins are added. The inactive compound may furthermore e.g. be a mineral which is chelated and thereby made available for the genetically transformed cells.

In contrast to traditional negative selection, in which the non-transformed cells are damaged or killed due to the presence of an antibiotic, herbicide or toxin in the substrate, inactive compounds or nutrients used in the positive selection method of the present invention have no direct adverse effect on the non-transformed cells. Instead, the transformed cells are provided with a physiological advantage allowing them to be identified and isolated, while the non-transformed cells are unaffected as such or less affected by the presence of the inactive compound or nutrient used for selection purposes.

The traditional "negative selection" methods are thus characterized by use of a selection gene which reduces the negative effect of an added compound on the transformed cells. In contrast the term "positive selection" as used in the context of the present invention refers to the use of a selection gene which produces or increases a positive effect of an added compound on the transformed cells.

Selection using this approach is thus achieved by making a compound available for the transformed cells, while the compound is not available or is less available for the non-transformed cells. The compound or nutrient in question, may be of the same type as any of those mentioned above, the difference being that the compound or nutrient in this case is transported into the transformed cells in addition to being activated in the transformed cells.

Examples of compounds which can exert a physiological effect upon entering the cell, but which are not easily taken up into the cell or a cell compartment, are strongly hydrophilic or hydrophobic compounds, in particular charged compounds, large molecules such as polymers, in particular proteins, peptides, oligo- and polysaccharides, including plant hormones, phosphorylated metabolites such as phosphorylated carbohydrates, phosphorylated vitamins, phosphorylated nucleosides, including cytokinins, and compounds which are conjugated to carboxylic acid-containing carbohydrates or amino acids, including plant hormone conjugates.

The "selective advantage" possessed by the transformed cells may be any difference or advantage with regard to the non-transformed cells which allows the transformed cells to be readily identified and isolated from the non-transformed cells. This will typically be a difference or advantage allowing the transformed cells to be identified by simple visual means, i.e. without the use of a separate assay to determine the presence of a marker gene.

When a polypeptide encoded by the co-introduced nucleotide sequence directly activates an inactive compound or nutrient in the transformed cells, the non-transformed cells may in certain cases contain or produce a certain amount of the polypeptide in question. For example, when the activating polypeptide is an enzyme, the non-transformed cells may contain a certain native enzyme activity, the native enzyme being of the same type as the introduced activating enzyme. In such cases the "inactive compound or nutrient" need not necessarily be completely inactive in the non-transformed cells, since it may be sufficient that the compound or nutrient is merely substantially less active in non-transformed cells than in transformed cells. In other words, a qualitative difference between the transformed cells and the non-transformed cells with regard to activation of the initially inactive compound or nutrient may in certain cases be sufficient for selection purposes. In such cases inhibitors or substrates which compete with the native enzymes may be added. Especially suitable are inhibitors activated by the native enzyme, resulting in self-catalyzed production of the active inhibitor to a level at which the native enzyme is substantially totally inhibited.

The activating polypeptide encoded by the co-introduced nucleotide sequence is not limited to any particular polypeptide and will of course be one which is active, either directly or indirectly, in relation to the particular compound or nutrient to be activated in the genetically transformed cells. The polypeptide is in particular often an enzyme.

One enzyme which has been found to be suitable for the selection of genetically transformed plant cells is β-glucuronidase ("GUS"), the selection being carried out using a glucuronide compound comprising a plant growth regulator which is cleaved by β-glucuronidase, e.g. a cytokinin glucuronide (the meaning of which will be apparent from the examples herein). It is surprising that selection of genetically transformed plant cells may be achieved using a GUS gene, since it has been found in connection with the present invention that higher plants possess native GUS activity. This finding is in contrast to that which has previously been reported. Thus, GB 2197 653-A states that higher plants contain no detectable β-glucuronidase activity and thereby implies that, since GUS activity is not found in higher plants, it is a relatively straightforward matter to monitor the expression of a gene construction of interest using the GUS gene. However, as explained below (see the Examples) this is not the case, and the use of a GUS gene to monitor the presence of a gene of interest is not at all simple or straightforward, due to the fact that higher plants do in fact contain a significant intrinsic (background) β-glucuronidase activity.

Thus, for the selection of genetically transformed plant cells the population of cells may be cultivated on or in a medium containing a cytokinin glucuronide which in the transformed cells is cleaved by the β-glucuronidase, thereby releasing free cytokinin and leading to shoot and/or callus induction in the transformed cells.

A number of novel cytokinin glucuronide compounds have been developed for the purposes of the present invention. The preparation of these compounds as well as their use for positive selection of genetically transformed cells is described in detail below.

In certain cases it may be desirable to modify the basic method disclosed herein, e.g. to provide a more effective selection or to simplify the selection procedure. When the inactive compound used for the positive selection process is one which is cleaved by β-glucuronidase, the basic method may be modified by various means to produce a better result. One of these means is the use of certain sterol glucuronide compounds, e.g. cholesteryl-β-D-glucuronide or β-sitosteryl-β-D-glucuronide, together with a sterol synthesis inhibiting compound such as tridemorph (4-tridecyl-2,6-dimethyl morpholine). Examples 5 and 6 below describe the use of such compounds. It is believed that by using a sterol synthesis inhibitor together with sterol glucuronides which counteract the effect of the sterol synthesis inhibitor upon hydrolysis by β-glucuronidase, so-called "cross feeding" (i.e. diffusion of the activated compound from the cell in which it is activated to another cell) during the selection process may be prevented, since the sterol compounds do not diffuse from cell to cell when the hydrophilic glucuronide moiety is cleaved off. Thus, a more localized effect is obtained. Corresponding results may be obtained with a large number of other glucuronides which contain a hydrophobic aglycone.

As explained above, it has in contrast to that which has previously been reported been found that higher plants do in fact possess native GUS activity. For this reason, the mere introduction of a GUS gene into a plant may not necessarily be sufficient to obtain the desired selection of the genetically transformed cells, and it may be necessary or desirable to reduce any native β-glucuronidase activity in the population of cells. Since an introduced β-glucuronidase may have different properties than a native β-glucuronidase, a reduction of any native β-glucuronidase activity may be accomplished in different ways, e.g. by addition to the culture medium of a β-glucuronidase inhibiting compound having more of an inhibiting effect on the native β-glucuronidase than on the β-glucuronidase encoded by the nucleotide or subsequence thereof. One such type of compound is an ammonium salt.

Native β-glucuronidase activity in the population of cells may also be substantially reduced by addition to the culture medium of a compound which upon hydrolysis results in a product which inhibits the activity of the native β-glucuronidase, and which preferably inhibits the activity of the native β-glucuronidase more than the activity of the introduced β-glucuronidase is inhibited. This may be performed in an autoregulated or localized manner, e.g. localized to specific compartments where the introduced GUS gene is located or is not located. An example of a hydrolysis product which inhibits native β-glucuronidase is glucuronic acid, e.g. resulting from the hydrolysis of glyccyrrhizic acid or steryl glucuronides.

Native β-glucuronidase activity in the population of cells may further be reduced by addition to the culture medium of a β-glucuronidase inhibitor, in particular a β-glucuronide which in cells without an introduced β-glucuronidase gene inhibits β-glucuronidase activity more than in cells with an introduced β-glucuronidase gene. This can e.g. be a poor β-glucuronidase substrate (a glucuronide) having a higher affinity for the native β-glucuronidase than for the introduced β-glucuronidase.

β-Glucuronidase encoded by the introduced β-glucuronidase gene used for the purposes of the present invention is active over a relatively broad pH range, while the native β-glucuronidase found in a variety of different plant species is only active within a relatively narrow range of pH values, typically about pH 4-5 (see Example 3 below). Native β-glucuronidase activity may therefore in this case be reduced by addition to the culture medium of a glucuronide which is able to be hydrolyzed by the native β-glucuronidase and which upon hydrolysis results in an increase in pH, e.g. o-coumaryl glucuronide.

Since it has been found that β-glucuronidase native to plants generally is active at a pH of about 4-5, the native β-glucuronidase activity may also be reduced by addition to the culture medium of a pH regulating compound which provides the culture medium with a pH of between about 5.5 and 8.5, preferably between about 6.0 and 8.0, e. g. between about 6.5 and 7.5, or a pH regulating compound which raises the pH in the cells or in compartments of the cells to a pH within these ranges. At these pH values β-glucuronidase encoded by the introduced GUS gene is active but native β-glucuronidase is substantially inactive. An example of a pH regulating compound which may be used is an ammonium salt or ammonium releasing compound, e.g. ammonium nitrate.

Finally, native β-glucuronidase activity may be reduced or substantially eliminated by a physical treatment such as a heat treatment, e.g. using a temperature in the range of 50-65°C in the form of a short pulse treatment of about 1-2 days before transfer to the selection substrate and/or using a temperature in the range of 30-45°C during selection (see Example 10).

Genetic transformation of plant cells is often performed using Agrobacterium strains, in particular strains of Agrobacterium tumifaciens. It has been found that certain dearmed Agrobacterium strains induce shoot formation due to the production of shoot-inducing substances during co-cultivation, and such strains should normally be avoided when GUS hydrolysis of cytokinin glucuronides is to be employed for the purposes of selection of genetically transformed cells. Thus, genetic transformation of the cells using a cytokinin glucuronide as the inactive compound is preferably performed using an Agrobacterium strain which does not produce cytokinins or other shoot (growth) inducing compounds, or which produces only an insubstantial amount of such compounds, thereby eliminating or substantially reducing induction of shoot growth due to the presence of living bacteria on or in the cells.

In certain cases, e.g. when an improved selection frequency is desired, it may be advantageous for the desired nucleotide sequence to be co-introduced with at least two different selection genes. The additional selection gene may be an additional gene coding for an enzyme (or other protein or polypeptide) suitable for positive selection according to the present invention, or it may be a gene coding for an enzyme (or other protein or polypeptide) suitable for traditional negative selection, e.g. coding for resistance to a toxin, antibiotic or herbicide. Thus, genetically transformed cells may be selected using a combination of positive selection and negative selection, the desired nucleotide sequence in the genetically transformed cells further being co-introduced with a subsequence coding for resistance to at least one toxin, antibiotic or herbicide, the medium comprising at least one toxic, antibiotic or herbicide to which the transformed cells are resistant.

As mentioned above, in one aspect of the present invention genetically transformed cells are disclosed which have been selected according to the above method, in particular plant cells, as well as plants, progeny or seeds derived from such genetically transformed plant cells. In particular, it is often an advantage that these cells are genetically transformed plant cells whose genome does not contain as a selection marker an introduced (i.e. non-native) nucleotide sequence coding for toxin, antibiotic or herbicide resistance. As explained above, there are concerns about whether it is safe to incorporate genes coding for e.g. antibiotic resistance in e.g. food plants. Genetically transformed plant cells selected by the method of the present invention which do not contain selection genes for e.g. antibiotic resistance, as well as plants, progeny and seeds derived from such cells, are therefore clearly advantageous from this point of view.

### Synthesis of compounds of the general formula I according to the invention

Various methods of greater or lesser generality are available for the synthesis of cytokinin glucuronides embraced by the general formula I, and two of what are believed to be best of these methods are described in the following:

A) Oxidation of the corresponding cytokinin D-glucoside. In this approach, the -CH₂OH group attached to the pyranose ring of the β-D-glucoside corresponding to the glucuronide in question is oxidized to a carboxyl function under appropriate conditions. Probably the best method of achieving this oxidation reaction in a straightforward and highly selective manner is catalytic oxidation by oxygen, suitably using platinum black or platinum-on-carbon as the catalyst and employing a weakly basic (pH 8-10) aqueous or aqueous alcoholic (such as aqueous ethanolic) reaction medium; the reaction may suitably be performed at a temperature in the interval 60-100°C for a period of time of from 2 to 24 hours. Oxidation (generally non-catalytic) by conventional oxidizing agents other than oxygen may also be applicable, but it is anticipated that the resulting oxidation reactions will, in general, give lower yields and be of poorer specificity [i.e. lead to mixtures of oxidation products - particularly unless measures are taken to protect other oxidizable functionalities of the glucoside starting material by the introduction of appropriate protecting groups (vide infra)].

An example of the catalytic oxidation approach is provided by Method 1 of Example 1C in the present application, in which N⁶-benzyladenine-9-β-D-glucopyranoside (BA9G) is oxidized to N⁶-benzyladenine-9-β-D-glucopyranuronic acid (which is subsequently isolated as the sodium salt) by oxidation with oxygen in the presence of platinum black.

This method appears to be of rather general applicability for the synthesis of cytokinin 9-glucuronides from corresponding 9-glucosides. It has also proved satisfactory for the synthesis of, for example, zeatin-O-glucuronide (cf. Example 1G herein) from the corresponding zeatin-O-glucoside; zeatin-O-glucuronide is an example of a compound of formula I according to the invention in which the glucuronide moiety is a substituent on an R⁶ moiety - as defined herein - of the C₂₋₈-alkenyl type, and the method in question is believed to be of rather broad generality for other cytokinin glucuronides according to the invention in which an O-glucuronide moiety resides as a substituent on an R⁶ group which is one of the following types as defined in connection with the general formula I: a benzyl group; a C₁₋₈₋alkyl or substituted C₁₋₈-alkyl; a C₂₋₈-alkenyl or substituted C₂₋₈-alkenyl.

This approach does not, however, appear to be generally applicable, for example, to the synthesis of cytokinin 3-glucuronides.

As already indicated, it is clear that when applying an approach involving oxidation of a cytokinin glucoside, any other oxidizable (under the oxidation conditions in question) functional groups which may be present in the starting cytokinin glucoside and which are to remain unchanged in the final glucuronide of formula I must be protected by the prior establishment of suitable protecting groups. Examples of potentially oxidizable groups which may be present in compounds within the definition of formula I are [with reference to their positioning as specified in connection with the general formula I (vide supra)]: an -SH group which may be present as an R² and/or R⁸ group; hydroxy or glucosyloxy groups which may be present as substituents on R⁶ groups of the substituted C₁₋₈-alkyl type or substituted C₂₋₈-alkenyl type; and ribosyl, 5'-phosphoribosyl or glucosyl groups which may be present as R⁹ or R⁷ groups. If, for example, it is necessary to protect aliphatic (alcoholic) hydroxy groups, such as hydroxy groups on secondary carbon atoms in ribosyl, 5'-phosphoribosyl or glucosyl groups, suitable protecting groups will often be, e.g., acetyl groups which may be introduced by methods which will be well known to a person skilled in the art and which may be removed, after the oxidation process, by alkaline hydrolysis.

However, when using the mild catalytic oxidation approach described above, hydroxy groups on primary aliphatic carbon atoms are, in general, susceptible to oxidation, whilst hydroxy groups on secondary (and tertiary) carbon atoms generally are not; thus, in oxidizing the -CH₂OH moiety in the 5-position of the glucopyranose ring of a cytokinin β-D-glucoside in this manner, the hydroxy groups at the 2-, 3- and 4-positions of the glucopyranose ring generally will not require protection. An -SH group which is to be present as a group R² or R⁸ will, however, generally require protection during this catalytic oxidation, and such an -SH group may suitably be protected as the benzyl derivative (vide infra in connection with method B described below).

Numerous appropriate cytokinin glucosides for use as starting materials in this method are commercially available; for example, a wide variety of these are obtainable from Apex Organics Ltd., Leicester, England, and some cytokinin 9-glucosides are obtainable from Sigma Chemical Company, P.O. Box 14508, St. Louis, MO 63178, USA. Cytokinin glucosides may also be prepared by established methods reported in the literature. For example, the synthesis of a variety of appropriate cytokinin 9-glucosides having R⁶ groups embraced within the present definition thereof may be achieved by straightforward extension of the method reported by Cowley et al. [Aust. J. Chem. 31 (1978) 1095] for the synthesis of 9-β-D-glucopyranosides of zeatin and N⁶-benzyladenine.

B) Syntheses of the Koenigs-Knorr type. This approach, which is based on the original method of W. Koenigs and E. Knorr [Chem. Ber. 34 (1901) 957], involves the reaction of methyl (2,3,4-tri-O-acetyl-α-D-glucopyranosyl bromide)-uronate [abbreviated MBTG; a suitable method for the preparation thereof is described by Bollenback et al., J. Amer. Chem. Soc. 77 (1955) 3310] with an alcoholic or phenolic hydroxy group, a mercapto (-SH) group or a ring nitrogen atom in an aromatic or unsaturated heterocyclic moiety

This approach probably provides the most generally applicable method for the synthesis of cytokinin glucuronides according to the invention (or of glucuronides of precursors which may subsequently readily be converted to the desired cytokinin glucuronides), starting from appropriate cytokinins [or cytokinin precursors (vide infra)], and it is believed to be of very broad applicability in the preparation of compounds embraced within the general formula I.

The general procedure is as follows: The appropriate cytokinin or cytokinin precursor (vide infra), dissolved in an solvent such as N,N-dimethylformamide (DMF), quinoline, propylene carbonate, methanol or diethyl ether, is allowed to react with a 1.25-2 molar equivalents of MBTG at a temperature in the range 25-100°C for a period of from 3 to 96 hours, preferably in the presence of an added halide (bromide) scavenger such as silver oxide or silver carbonate (when employing, for example, DMF as solvent, the solvent itself often functions adequately as halide scavenger, in which case the addition of a further scavenger is not essential). This reaction yields the methyl ester of the intermediate peracetylated glucuronide, which is generally isolated and purified; this may suitably be accomplished, for example, (i) by evaporation of the solvent, followed by extraction of the residue with a solvent such as chloroform, removal of the latter solvent from the extract, and purification of the resulting crude intermediate by recrystallisation and/or conventional column chromatographic techniques; or, for example, (ii) by separation of the intermediate from the reaction mixture by conventional column chromatography carried out directly on the reaction mixture, followed by removal of the elution solvent from the eluted fraction(s) of interest and recrystallisation of the crude product.

In cases where the desired end-product of formula I is to have the glucuronide moiety in the amide (glucuronamide) form, conversion of the peracetylated methyl ester form of the glucuronide moiety to the amide form is suitably carried out at this stage, and this may generally be accomplished by treating the peracetylated methyl ester (preferably purified, e.g. in the manner outlined above) with a solution of anhydrous ammonia in anhydrous methanol at low temperature, e.g. a temperature between 0°C and -10°C, or - as an alternative possibility - with a concentrated (suitably saturated) aqueous solution of ammonia at approximately ambient temperature, for a period of 0.5-4 hours. The glucuronamide may then be isolated by evaporation of the ammonia solution, e.g. under vacuum, and recrystallisation from an appropriate solvent or solvent mixture, such as 90% aqueous ethanol. An example of this conversion is provided by Example 1 B herein, in which N⁶-benzyladenine-3-glucuronamide (BA3GNamide) is prepared from the corresponding methyl ester.

Furthermore, in the case of procedures starting with certain types of cytokinin precursors, a chemical transformation which is necessary in order to convert the cytokinin precursor moiety to the appropriate cytokinin moiety may often be performed on the methyl ester before proceeding to liberate the free glucuronide. As an example, the synthesis of cytokinin 9-glucuronides (whose synthesis by a catalytic oxidative approach has already been described above) may normally be accomplished satisfactorily starting from a substituted or unsubstituted purine having a chlorine atom in the 6-position; the latter 6-chloro compound may, in general, be converted to the methyl ester of the corresponding peracetylated 9-glucuronide by means of the above-described general procedure using MBTG, and the 6-chloro group may then be suitably be converted to the desired -NH- R⁶ moiety by allowing the product from the latter reaction to react with the corresponding amine (R⁶-NH₂), which generally may suitably be generated in situ from the amine hydrochloride (R⁶-NH₂·HCl) and an excess (suitably a 2-4 fold molar excess) of an appropriate base, e.g. a tertiary aliphatic amine such as triethylamine, in a suitable polar solvent, such as a C₁₋₄ aliphatic alcohol, at an temperature in the range 65-120°C. This is illustrated by Method 2 of Example 1 C herein, in which the synthesis of N⁶-benzyladenine-9-glucuronide (BA9GN) (as its sodium salt) by this method is described.

The acetyl groups on the glucuronide moiety are then removed by base hydrolysis using a base such as aqueous sodium hydroxide, aqueous methanolic or ethanolic sodium hydroxide, or methanolic ammonia at a temperature in the range 0-25°C for a period of from 0.5 to 6 hours. Using a base such as one of the above-mentioned aqueous or alcoholic sodium hydroxide solutions, this procedure gives - after neutralization of the excess of base - the salt form of the corresponding cytokinin glucuronide, whereas the use of a reagent such as methanolic ammonia and subsequent removal of excess ammonia by evaporation leads to the amide form of the glucuronide. The latter is suitably purified by conventional means, such as chromatography, particularly reverse-phase chromatography, and/or recrystallisation from a suitable solvent, such as an aqueous organic solvent, e.g. 80-90% aqueous ethanol.

Clearly, if the starting cytokinin or cytokinin precursor contains one or more functionalities which are capable of reaction with MBTG under the reaction conditions in question, and which are to be present unchanged in the final glucuronide of formula I, then such functionalities must be protected by the prior establishment of suitable protecting groups; examples of such reactive functionalities which may be present in starting cytokinins leading to compounds within the definition of formula I are [with reference to their positioning as specified in connection with the general formula I (vide supra)]: -OH of -SH present as an R² group; -OH or -SH present as an R⁸ group; -OH or -NH₂ present as a substituent on the phenyl ring of an R⁶ group of the substituted benzyl type; -OH or glucosyloxy groups present as substituent(s) on an R⁶ group of the substituted C₁₋₈-alkyl type or substituted C₂₋₈-alkenyl type; ribosyl, 5'-phosphoribosyl or glucosyl groups which may be present as R⁹ or R⁷ groups; and -NH₂ in a -CH₂CH(NH₂)COOH group present as an R⁹ group.

With respect to protecting groups suitable for protection of the above-mentioned examples of reactive functionalities which may be present in starting cytokinins, an -OH group may generally suitably be protected by the introduction of an acetyl group (vide supra in connection with the oxidative method of preparation of compounds of formula I) so as to form the corresponding acetoxy (-OOCCH₃) group. An -SH group may generally very suitably be protected as the benzylthioether (-SCH₂C₆H₅) derivative by the introduction of a benzyl group [e.g. by reaction with benzyl chloride in a manner similar to that described in further detail below in connection with cytokinin precursors which contain, at least formally, oxo (=O) or thioxo (=S) groups at the 2-position, and an oxo group at the 6-position (vide infra)]. An -NH₂ group may generally suitably be protected by conversion to a phthalimido group as follows: the cytokinin or cytokinin precursor is heated with an excess of phthalic anhydride in a relatively inert solvent, such as chloroform or 1,2-dimethoxyethane, at a temperature in the region 70-100°C for a period of hours, often suitably about 4 hours. The -NH₂ group may, after performance of the Koenigs-Knorr type reaction, subsequently be regenerated by treatment with an aqueous alcoholic (such as an aqueous ethanolic) solution of hydrazine.

A group of cytokinin precursors which are particularly well suited for use in the synthesis of cytokinin glucuronides according to the invention having the glucuronide moiety (corresponding to R¹⁰ in the general formula I) attached as -O-R¹⁰ or -S-R¹⁰ at the 2-position of the purine ring system are cytokinin precursors which contain, at least formally, oxo (=O) or thioxo (=S) groups at the 2-position, and an oxo group at the 6-position; it should be mentioned here that compounds of the types in question having 2-oxo and 2-thioxo groups will, at least in solution, generally be in tautomeric equilibrium with the corresponding 2-hydroxy and 2-mercapto compounds (the 6-oxo group then being present as a 6-hydroxy group), and the latter tautomeric forms will, in general, be present in a significant proportion.

The latter-mentioned hydroxy or mercapto group at the 2-position is converted, in the final phase of the overall synthesis procedure, by means of the Koenigs-Knorr type procedure to the corresponding -O-R¹⁰ or -S-R¹⁰ group, respectively; however, before performing the Koenigs-Knorr type reaction sequence the 6-hydroxy group is suitably converted, via its intermediate conversion to a 6-halo (preferably 6-chloro) group, to the -NH-R⁶ moiety shown in formula I, and this generally requires that the hydroxy or mercapto group at the 2-position is protected by a suitable protecting group during this conversion sequence. For both these groups, the protecting group of choice is a benzyl group, which may normally be introduced straightforwardly by a reaction involving the gradual addition of a slight excess of benzyl chloride to a stirred solution or suspension of the cytokinin precursor in question in aqueous base (pH typically about 12-13), such as aqueous sodium or potassium hydroxide, at approximately ambient temperature.

After continued stirring for a period of, typically, 1-2 hours, the reaction mixture is neutralized by addition of, e.g., glacial acetic acid, and the insoluble, benzyl-protected product is isolated by filtration.

The 6-hydroxy group of the resulting 2-benzyloxy- or 2-benzylthio-6-purinol derivative is then converted to a 6-chloro group, suitably using an excess of a chlorinating reagent such as phosphorus oxychloride (phosphoryl chloride) in the presence of an organic base, such as N,N-diethylaniline; the latter reaction will normally suitably be performed under reflux conditions for a period of from about 10 minutes to about 3 hours.

The 6-chloro group may then be suitably be converted to the desired -NH-R⁶ moiety by allowing the product from the latter reaction to react with the corresponding amine, R⁶-NH₂, which generally may suitably be generated in situ from the amine hydrochloride (R⁶-NH₂-HCl) and an excess (suitably a 2-4 fold molar excess) of an appropriate base, e.g. a tertiary aliphatic amine such as triethylamine, in a suitable polar solvent, such as a C₁₋₄ aliphatic alcohol (e.g. 1-butanol), at an temperature in the range 65-120°C.

After isolation of the product, the protecting group is removed to regenerate the free 2-hydroxy or 2-mercapto group; in the case of a benzyl protecting group, this may suitably be accomplished, for example, by treatment of the product with an excess of sodium in liquid ammonia.

In the case of products having a 2-mercapto group at this stage, it is believed to be generally advantageous to convert the 2-mercapto group to a salt form (-S-), e.g. the potassium salt form, before proceeding to introduce the glucuronide moiety by means of the Koenig-Knorr type procedure described above. An example of a suitable procedure for this purpose is described in step 4 of working Example 1E herein, and the conditions described there are believed to be of rather general applicability. It may, however, in some cases be possible to perform the Koenigs-Knorr type reaction with MBTG (see below) directly without prior conversion of the 2-mercapto group to the salt form.

Finally, the glucuronide moiety is introduced by reaction with MBTG as described above in connection with the Koenigs-Knorr type procedure.

An example illustrating the sequence of synthesis steps described above is given in Example 1E for the synthesis of the sodium salt of N⁶-(2'-isopentenyl)adenine-2-thioglucuronide (IP2SGN) starting from 2-thioxanthine, and much of the further information provided therein with regard to choice of recrystallisation and extraction solvents, choice of concentrations and amounts of reagents, etc., is believed to be of more general applicability in performing the sequence of reactions outlined above starting from a cytokinin precursor having a "2-thioxo" group (i.e. a 2-mercapto group) and a "6-oxo" group (i.e. a 6-hydroxy group).

Similarly, a group of cytokinin precursors which are particularly well suited for use in the synthesis of cytokinin glucuronides according to the invention having the glucuronide moiety (corresponding to R¹⁰ in the general formula I) attached as -O-R¹⁰ or -S-R¹⁰ at the 8-position of the purine ring system are cytokinin precursors which contain, at least formally (for reasons similar to those outlined above in connection with cytokinin precursors having a formal 2-oxo or 2-thioxo group and a formal 6-oxo group), an oxo (=O) or thioxo (=S) group at the 8-position, and which further have a hydroxy group at the 6-position. These may, in general, be converted to the desired end-product of formula I using a sequence of reactions (protection, introduction of a 6-chloro group, introduction of the -N-R⁶ moiety, deprotection, and finally reaction with MBTG) analogous to that outlined above for cytokinin glucuronides having the glucuronide moiety attached as -O-R¹⁰ or -S-R¹⁰ at the 2-position of the purine ring system. An example of this is provided by Example 1 F herein for the synthesis of N⁶-(2'-isopentenyl)-adenine-8-thioglucuronide (as the sodium salt thereof).

Compounds of the formula I in which the glucuronide moiety is in the carboxylic acid form may, in general, be prepared from the corresponding salt form, e.g. sodium salt form (prepared in a manner already outlined above) by acidifying a stirred solution or solution/suspension of the salt form in an appropriate solvent (e.g. an aqueous alcohol, such as aqueous ethanol), preferably at a temperature below 25°C, with a mineral acid such as hydrochloric acid to a pH of about 2.5. After stirring for a few minutes, the solvent is then removed under vacuum. The crude residue may advantageously be subjected to a chromatographic treatment to remove inorganic salts (see, e.g., Example 1I herein for details of a typical procedure and a suitable chromatographic substrate for this purpose), after which the product may be recrystallised from an appropriate solvent, typically a polar solvent such as methanol or ethanol.

Compounds of the formula I in which the glucuronide moiety is in the methyl ester or ethyl ester form may generally very suitably be prepared, in high yield, by reaction of the corresponding carboxylic acid form of the cytokinin glucuronide (prepared, for example, as described above) with diazomethane or diazoethane, respectively, using reaction conditions which are normal for this type of reaction and which will be well known to a person skilled in the art.

Numerous cytokinins and cytokinin precursors appropriate for use as starting materials in connection with method B), above, are commercially available, while others may be synthesized by established methods reported in the literature. For example, the synthesis of a variety of appropriate N⁶-substituted adenines having R⁶ groups embraced within the present definition thereof may be achieved by literature methods referred to by Iwamura et al. [Phytochemistry 19 (1980) 1309], and numerous 2-, 8- and 2,8-substituted N⁶-(2'-isopentenyl)adenines having R² and/or R⁸ groups embraced within the present definition thereof may be prepared as described by Dammann et al. [Phytochemistry 13 (1974) 329].

Currently preferred compounds of the formula I are the following:
a compound of formula I wherein R² is H, R³ is a β-D-glucopyranuronosyl group or a salt thereof at the carboxylic acid function, R⁹ and X are half-bonds which together form a bond, R⁶ is benzyl, R⁷ and Y are half-bonds which together form a bond, and R⁸ is H;
a compound of formula I wherein R² is H, R³ is the amide derivative of β-D-glucopyranuronosyl at the carboxylic acid function thereof, R⁹ and X are half-bonds which together form a bond, R⁶ is benzyl, R⁷ and Y are half-bonds which together form a bond, and R⁸ is H;
a compound of formula I wherein R² is H, R³ and X are half-bonds which together form a bond, R⁹ is a β-D-glucopyranuronosyl group or a salt thereof at the carboxylic acid function, R⁶ is benzyl, R⁷ and Y are half-bonds which together form a bond, and R⁸ is H;
a compound of formula I wherein R² is H, R³ is a β-D-glucopyranuronosyl group or a salt thereof at the carboxylic acid function, R⁹ and X are half-bonds which together form a bond, R⁶ is 2-isopentenyl, R⁷ and Y are half-bonds which together form a bond, and R⁸ is H;
a compound of formula I wherein R² is an -S-β-D-glucopyranuronosyl group or a salt thereof at the carboxylic acid function, R⁹ is H, R³ and X are half-bonds which together form a bond, R⁶ is 2-isopentenyl, R⁷ and Y are half-bonds which together form a bond, and R⁸ is H;
a compound of formula I wherein R² is H, R⁹ is H, R³ and X are half-bonds which together form a bond, R⁶ is 2-isopentenyl, R⁷ and Y are half-bonds which together form a bond, and R⁸ is an -S-β-D-glucopyranuronosyl group or a salt thereof at the carboxylic acid function; and
a compound of formula I wherein R² is H, R³ and X are half-bonds which together form a bond, R⁹ is H, R⁶ is a group of the formula 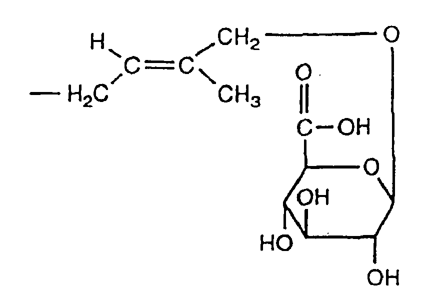
or a salt thereof, R⁷ and Y are half-bonds which together form a bond, and R⁸ is H.

### Synthesis of compounds of the general formula II according to the invention

Compounds of formula II may be prepared straightforwardly using the easily prepared methyl ester of o-coumaric acid (i.e. 3-(2-hydroxyphenyl)-2-propenoic acid) as starting material. The latter ester may be prepared by heating the free acid (obtained, for example, from Aldrich Chemical Company, Gillingham, Dorset, England, under catalogue number H2,280-9) under reflux in methanol in the presence of sulfuric acid. It should be noted here that a mixture of the cis- and trans-forms (with respect to the ethylenic double bond) of the moiety is obtained. It is believed that the trans-form, irrespective of the method of preparation, is generally predominant initially. However, this does not matter, since it has been found that both the cis (coumarinyl) and trans (coumaryl) glucuronides are ultimately converted to coumarin after GUS hydrolysis, due to the fact that coumaric acid is slowly converted (non-enzymatically; presumably via catalysis by light) to coumarin over a period of a few days.

Efficient procedures for the synthesis of the compounds of formula II in which (a) R¹ and R¹⁰ are in the carboxylic acid form and (b) R¹ and R¹⁰ are in the amide form are described in detail in Examples 1H and 1I, respectively, herein. The compound(s) of formula II in which both R¹ and R¹⁰ are in the sodium salt form may suitably be obtained by base hydrolysis of the methyl ester of the peracetylated glucuronide [prepared (from methyl o-coumarate) and isolated as in the first part of the synthesis procedure described in Example 1I] using methanolic sodium hydroxide in the manner described at the beginning of the second part of the synthesis procedure described in Example 1I; instead of adjusting the pH of the hydrolysate to 2.5 with hydrochloric acid as described in Example 1I, the mixture is merely neutralized (to pH ca. 7) with hydrochloric acid. The sodium salt form is suitably isolated from the mixture by chromatography on, e.g., Amberlite XAD-2 non-ionic resin, the column being washed with water to remove inorganic salts and then eluted with, typically, methanol. The crude glucuronide sodium salt form obtained by removal of the methanol may then suitably be recrystallised from, for example, absolute methanol. The potassium salt form will clearly also be preparable by a closely analogous procedure using, e.g., methanolic potassium hydroxide in the hydrolysis procedure.

Compounds of formula II in which R¹ and R¹⁰ are both in the methyl ester form or both in the ethyl ester form may suitably be prepared by reaction between diazomethane or diazoethane, respectively, and the compound(s) of formula II in which both R¹ and R¹⁰ are in the carboxylic acid form. The reaction conditions herefor will suitably be as described above for the analogous preparation of methyl or ethyl ester forms of cytokinin glucuronides.

Currently preferred compounds of the general formula II are the following:
a compound of formula II wherein R¹ is cis- and/or trans-2-amidoethenyl [cis- and/or trans-CH=CHCONH₂], and R¹⁰ is the amide derivative of β-D-glucopyranuronosyl at the carboxylic acid function thereof (2-hydroxycinnamyl-β-D-glucopyranuronamide).
a compound of formula II wherein R¹ is cis-2-carboxy ethenyl [cis -CH=CHCOOH], and R¹⁰ is a β-D-glucopyranuronosyl group (2-hydroxycinnamyl-β-D-glucopyranuronic acid).

The Examples below illustrate the general principles of the present invention in plants, in particular using a β-glucuronidase as a selection gene and using novel glucuronide substrates which are able to be hydrolyzed by this gene. On the basis of this work, it is contemplated that genes such as the β-glucuronidase gene may be used for a number of related purposes. Thus, the β-glucuronidase gene may be employed in a method for obtaining a localized or tissue specific plant growth regulating effect in a plant part or plant tissue which expresses an introduced β-glucuronidase gene at a higher level than other parts or tissues in the same plant, the method comprising subjecting the plant to a compound which is capable of being hydrolyzed by the introduced β-glucuronidase gene, so that the compound is hydrolyzed in the part or tissue containing the introduced β-glucuronidase gene, thereby releasing a growth regulating compound in the tissue and leading to a growth regulating effect only in this part or tissue or leading to a growth regulating effect is this part or tissue which is greater than the effect obtained in other parts or tissues in the plant. It is also contemplated that it may be advantageous to employ plant growth regulators such as cytokinins in the form of glucuronides or glucuronide derivatives rather than as free cytokinins, e.g. in order to take advantage of the fact that they would presumably be transported and distributed differently in plants as compared to e.g. the corresponding free or ribosylated cytokinins.

Similarly, the Examples below suggest certain other uses for β-glucuronidase. One of these is in an in vitro method for screening and identifying cytokinin glucuronide compounds (or glucuronide compounds of other plant growth regulators) which are capable of being hydrolyzed in vivo by an introduced β-glucuronidase gene. β-Glucuronidase may also be used in a system for screening for compounds which are suitable for use as selection agents in the positive selection method disclosed herein (see Example 2). In Examples 3 and 12 systems are described which may be used for screening for compounds which selectively inhibit a native β-glucuronidase enzyme in plant cells without substantially affecting the activity of an enzyme encoded by an introduced β-glucuronidase gene.

### EXAMPLE 1

### SYNTHESIS OF GLUCURONIDE COMPOUNDS

The synthesis of a number of novel glucuronide compounds is described below. In addition to the abbreviations given for each of the individual synthesized compounds, the following abbreviations are used:
- BA: N⁶-benzyladenine
- AcOH: acetic acid
- DMF: N,N-dimethylformamide
- IP: N⁶-(2-isopentenyl)adenine
- MBTG: methyl(2,3,4-tri-O-acetyl-α-D-glucopyranosyl bromide) uronate

### EXAMPLE 1A

3-β-D-glucopyranuronosyl-6-benzylaminopurine, sodium salt
- Synonyms:: N⁶-benzyladenine-N³-β-D-glucopyranuronic acid, sodium salt,
N⁶-benzyladenine-3-glucuronide, sodium salt
- Abbreviation:: BA3GN sodium salt

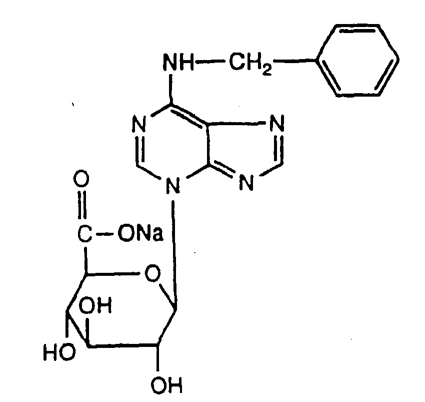

### Condensation of N⁶-benzyladenine (BA) and methyl (2,3,4-tri-O-acetyl-α-D-glucopyranosyl bromide)uronate (MBTG)

BA (12.6 mmole) and MBTG (15.1 mmole) (Bollenback et al., 1955, J. Amer. Chem. Soc., Vol. 77, pp. 3310-3315) were suspended in anhydrous N,N-dimethylformamide (DMF) (50 ml) and heated at 100°C for approximately 10 hours. Most of the DMF was removed under vacuum and the crude product was dissolved in chloroform (300 ml) and partitioned with water (3 x 300 ml). After drying over anhydrous magnesium sulphate the chloroform extract was evaporated under vacuum and a dark syrup was recrystallised from ethanol. The crude product (a mixture of BA, BA9GN and BA3GN as their peracetylated methyl esters) was purified over 100 g silica packed in chloroform eluted with a gradient of 0-4% ethanol in chloroform. Crude peracetyl BA3GN methyl ester was recrystallised from ethanol. Yield: 280 mg of a colourless, amorphous solid.

Peracetyl BA3GN methyl ester was hydrolysed by treatment with 5% sodium hydroxide in 50% aqueous ethanol at room temperature. Five minutes after the solid had dissolved the reaction mixture was carefully neutralised with hydrochloric acid while cooling on ice. After drying under vacuum, crude BA3GN sodium salt was purified by reverse-phase chromatography (over 100 g octadecylsilica) eluting with water (11) followed by 20% aqueous methanol followed by recrystallisation from ethanol. Pure BA3GN sodium salt (150 mg) was obtained as a colourless, microcrystalline solid which was dried to constant weight over calcium chloride under vacuum.

### Analysis

| UV | | |
|---|---|---|
| Ethanol | A max | 297 nm |
| Ethanol/acetic acid | A max | 291 nm |
| Ethanol/ammonia | A max | 297 nm |

These values are identical to the literature values for BA-3-β-D-glucopyranoside and N³, N⁶-disubstituted adenine. (N J Leonard, K L Carraway and J P Helgeson, J. Heterocyclic Chem. 1965, 2, 291-297).

### HPLC

HPLC was performed using a 10 x 0.46 cm column of octadecyl silica, eluting isocratically with 60% methanol containing 10% acetic acid at 1 ml/min. UV monitor at 290 nm.

The BA3GN sodium salt had a purity of 95+% and a content of free BA estimated as <0.05%.

### Hydrolysis by β-glucuronidase (GUS)

BA3GN sodium salt (500 µg) in 500 µl of 50 mM sodium phosphate buffer, pH 7.0 was incubated with β-glucuronidase (GUS, Sigma Type G7896), 2500 Sigma "Fishman" units for 18 hours at 37°C. HPLC (conditions as above) showed virtually complete removal of the BA3GN peak with the production of a peak which co-chromatographed in 1:1 mixture with authentic BA. This confirms the identity of the product as a conjugate of BA and β-D-glucuronic acid.

The following analysis was obtained for another portion of BA3GN sodium salt prepared as described above:

| UV | | |
|---|---|---|
| Ethanol | A max | 297 nm |

### HPLC

15 x 0.46 cm octadecyl silica, eluted with a gradient of 20-60% methanol/10% acetic acid over 30 min. at 1 ml/min. HPLC purity 99.5%. Free BA content < 0.05%.

### TLC

Silica developed with 1-butanol/acetic acid/water (12/3/5). TLC purity 99.5%. Free BA content < 0.05%.

### Hydrolysis by β-glucuronidase (GUS)

BA3GN sodium salt (500 µg) in 500 µl of 50 mM sodium phosphate buffer, pH 7.0 was incubated with β-glucuronidase (GUS, Sigma Type G7896), 2500 Sigma "Fishman" units for 12 hours at 37°C. HPLC and TLC showed virtually complete (> 99%) removal of the BA3GN to yield a compound which co-chromatographed in a 1:1 mixture with authentic BA.

### EXAMPLE 1B

3-β-D-glucopyranuronamido-6-benzylaminopurine
- Synonyms:: N⁶-benzyladenine-N³-β-D-glucopyranuronamide N⁶-benzyladenine-3-glucuronamide
- Abbreviation:: BA3GNamide

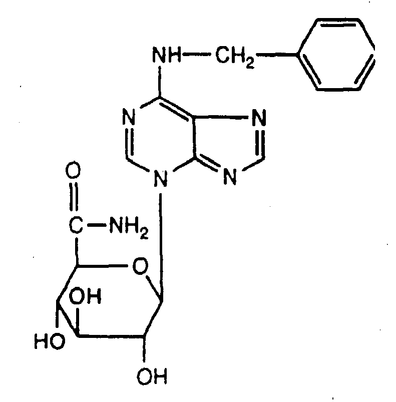

### Synthesis

Recrystallised peracetyl BA3GN methyl ester (1.5 g) prepared as in Example 1 A, was suspended in anhydrous methanol (200 ml) and cooled to 0°C. Further anhydrous methanol (400 ml), saturated with anhydrous ammonia at -10°C, was added and the mixture stirred on ice. Further ice-cold anhydrous methanol was added until the solid dissolved completely. The reaction mixture was stirred on ice for a further 3 h. Excess ammonia and methanol were removed under vacuum and the product was triturated thoroughly with hot ethanol to yield a colourless solid (1.3 g).

BA3GNamide, as with other adenine-3-glycosides, is only sparingly soluble in water or alcohol but dissolves at 2.4 mg/ml in 50% aqueous methanol containing 25% acetic acid.

### Analysis

### TLC (silica-chloroform/methanol/glacial acetic acid, 50/50/5)

Single UV spot (Rf 0.36) with no detectable impurities at 50 µg loading. Purity 98+%. No detectable (< 1%) peracetyl BA3GN methyl ester (Rf 0.89) or BA3GN (Rf 0.02).

### TLC (silica-chloroform/methanol, 9/1)

Used to test for BA content. BA3GNamide at 192 µg loading showed no detectable 6-benzyladenine. BA content therefore < 0.2%.

### EXAMPLE 1C

9-β-D-glucopyranuronosyl-6-benzylaminopurine, sodium salt
- Synonyms:: N⁶-benzyladenine-N⁹-β-D-glucopyranuronic acid, sodium salt
N⁶-benzyladenine-9-glucuronide, sodium salt
- Abbreviation:: BA9GN sodium salt

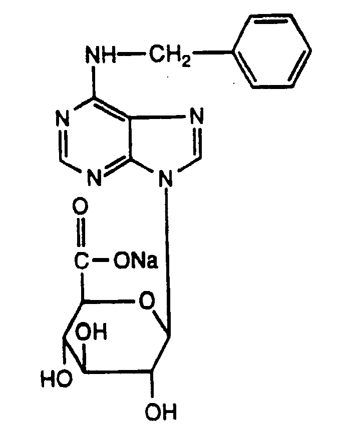

### Synthesis

### Method 1

### Catalytic oxidation of N⁶-benzyladenine-9-β-D-glucopyranoside (BA9G)

BA9G (50 mg) was suspended in 50 mM sodium bicarbonate (25 ml) with platinum black (200 mg). The mixture was heated at 80°C on a water bath while oxygen was passed through vigorously. A further 100 mg of platinum catalyst was added after 4 hours. Approximately 95+% of BA9G was converted to the corresponding 9-β-D-glucopyranuronic acid and some BA after 20 hours of treatment. The mixture was neutralised and the product BA9GN sodium salt was purified by reverse-phase chromatography (over 20 g octadecylsilica) eluting with 200 ml water followed by 20% MeOH.

Pure BA9GN sodium salt, free of glucoside and BA, was dried to a colourless solid over calcium chloride under vacuum.

### Method 2

### Condensation of 6-chloropurine and methyl (2,3,4-tri-O-acetyl-α-D-glucopyranosyl bromide)uronate (MBTG)

6-Chloropurine (dried over phosphorus pentoxide, 1.13 g), MBTG (3.87 g) and freshly-dried potassium carbonate (1.5 g) were stirred in anhydrous propylene carbonate (30 ml) at room temperature for 24 hours. The dark mixture was filtered and purified by column chromatography over silica (100 g) eluting with a 0-80% gradient of ethyl acetate in chloroform. The main fraction (other than unreacted 6-chloropurine) was dried and recrystallised from boiling ethanol to yield methyl 6-chloropurine-9-(2',3',4'-tri-O-acetyl-β-D-glucopyranuronate) as a pale yellow solid (450 mg), which was dried over phosphorus pentoxide under vacuum. HPLC indicated a purity of 95+%.

Methyl 6-chloropurine-9-(2',3',4'-tri-O-acetyl-β-D-glucopyranuronate (312 mg) and benzylamine (171 µl) were heated together in 1-butanol (13.5 ml) at 100°C for 1 hour. The solid dissolved readily to give a clear yellow solution. Most of the butanol was removed under vacuum to yield a whitish solid which was shaken with 5% sodium hydroxide in 50% aqueous ethanol (25 ml) for 1-2 hours at room temperature. After neutralization the product was dried under vacuum to remove traces of butanol and crude BA9GN sodium salt was purified by reverse-phase chromatography as in Method 1.

The pure product was dried under vacuum over calcium chloride and phosphorus pentoxide to yield a colourless solid (210 mg) which co-chromatographed with BA9GN prepared by catalytic oxidation.

### Analysis of BA9GN sodium salt prepared by condensation reaction, Method 2

| UV | | | |
|---|---|---|---|
| 95% Ethanol | A max | 270 nm | (17,400) |
| 95% Ethanol/0.1 M HCl | A max | 270 nm | (16,200) |
| 95% Ethanol/0.1 M NaOH | A max | 270 nm | (17,400) |

These results are consistent with the structure of an N⁶, N⁹-disubstituted adenine. The extinction coefficients are virtually the same as pure BA9G indicating freedom from non-UV absorbing contaminants. UV purity 95+%.

### HPLC

10 x 0.46 cm octadecylsilica column, UV monitor at 270 nm. Isocratic (50% methanol/0.2 M acetic acid, 2 ml/ min.) and gradient HPLC (0-60% methanol/0.2 M acetic acid, 2 ml/min.) show a sharp, symmetrical peak for BA9GN, which elutes just before the corresponding glucoside. BA9GN prepared by condensation reaction co-chromatographs in a 1:1 mixture on HPLC (gradient and isocratic) with BA9GN prepared by catalytic oxidation. This confirms that BA9GN prepared by the condensation reaction is the β-D-glucopyranosyl isomer. BA9GN contains no detectable (< 2%) α-anomer or other impurities, including BA. HPLC purity of BA9GN 98+%.

### TLC (silica-chloroform/methanol, 9/1)

Used to measure BA contamination of BA9GN product. BA9GN does not move from origin in this system. Minimum detection limit of BA = 200 ng. BA9GN at 200 µg loading shows no detectable BA or other contaminants. TLC purity 98+%. BA content < 0.1%.

### Acid hydrolysis

Mineral acid converts cytokinin glucosides to the corresponding free cytokinin base. Treatment of BA9GN sodium salt (1 mg/ml) with 1 M hydrochloric acid at 100°C overnight produced a single spot on TLC which co-chromatographed with authentic BA. This test confirmed that BA9GN was a acid-labile conjugate of BA.

### Enzymatic hydrolysis

BA9GN sodium salt (500 µg) in 500 µl of 50 mM sodium phosphate buffer, pH 7.0 incubated with Sigma β-glucuronidase (GUS, Type G7896), 2500 "Fishman" units for 18 hours at 37°C. HPLC and TLC showed no detectable production of BA. Further incubation at room temperature for 3 days showed no hydrolysis. BA9GN is therefore not susceptible to β-glucuronidase hydrolysis.

### EXAMPLE 1D

3-β-D-glucopyranuronosyl-6-(3-methyl-but-2-enylamino)purine, sodium salt
- Synonyms:: N⁶-(2'-isopentenyl)adenine-N³-β-D-glucopyranuronic acid, sodium salt
N⁶-(2'-isopentenyl)adenine-3-glucuronido, sodium salt
- Abbreviation:: IP3GN sodium salt

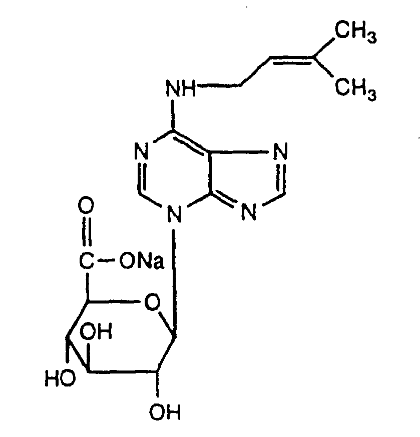

### Synthesis

N⁶-(2-isopentenyl)adenine (9.48 g) and MBTG (22.1 g) were heated in anhydrous DMF (170 ml) at 100°C for 12 h. Most of the DMF was removed under vacuum on a boiling water-bath and the cooled syrup taken up in chloroform (500 ml). The chloroform solution was extracted with water (3 x 500 ml) and the organic extract dried over anhydrous sodium sulphate. Most of the chloroform was removed under vacuum and the syrup chromatographically-purified over silica developed with a gradient of 0 to 3.75% methanol in chloroform. Crude peracetyl IP3GN methyl ester was recrystallised from methanol with charcoal decolourisation to yield pure, colourless peracetyl IP3GN methyl ester (3.2 g) which was dried under vacuum over calcium chloride. A portion of peracetyl IP3GN methyl ester (1.2 g) was hydrolysed by dissolving in approximately 1 l of 75% aqueous methanol containing 5% sodium hydroxide and stirring the mixture for 10 min at room temperature. The mixture was cooled on ice, carefully neutralised with hydrochloric acid and reduced to a syrup under vacuum. The crude product was purified by successive chromatography over XAD-2 resin and octadecylsilica to yield IP3GN sodium salt as a colourless, microcrystalline solid which was dried over calcium chloride (810 mg).

IP3GN sodium salt is soluble in water at 2 mg/ml.

### Analysis

### TLC (silica-1-butanol/glacial AcOH/water, 12/3/5

IP3GN sodium salt gives a single, sharp spot (Rf = 0.32) at 100 µg loading with no detectable N⁶-(2-isopentenyl)adenine (IP) (Rf = 0.66) or other contaminants. Purity 99.5+%.

### Hydrolysis by β-glucuronidase (GUS)

IP3GN sodium salt (500 µg) in 500 µl of 50 mM sodium phosphate buffer, pH 7.0 was incubated with β-glucuronidase (GUS, Sigma Type G7896), 2500 Sigma "Fishman" units for 12 hours at 37°C. TLC showed removal of the UV spot corresponding to IP3GN with the production of a new UV spot which co-chromatographed with authentic IP.

### EXAMPLE 1E

6-(3-methyl-but-2-enylamino)purine-2-yl-1-thio-β-D-glucopyranuronic acid, sodium salt
- Synonyms:: N⁶-(2'-isopentenyl)adenine-2-thioglycopyranuronic acid sodium salt
N⁶-(2'-isopentenyl)adenine-2-thioglucuronide, sodium salt
- Abbreviation:: IP2SGN sodium salt

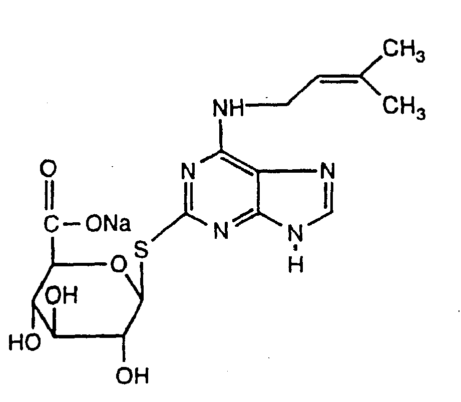

### Synthesis

### 1. 2-Benzylthio-6-purinol

Benzyl chloride (1.4 ml) was added dropwise with vigorous stirring to a solution of 2 g of 2-thioxanthine in 12 ml of 1 M sodium hydroxide, diluted to 140 ml with water. After addition was complete a cream-coloured precipitate formed. The reaction mixture was stirred for an additional hour at room temperature and filtered. The solid was washed thoroughly with water and dried overnight under vacuum over calcium chloride and to constant weight over phosphorus pentoxide. The crude product (2 g) was used directly in the next step without recrystallisation.

### 2. 2-Benzylthio-6-chloropurine

2-Benzylthio-6-purinol (2 g) was covered with a mixture of phosphorus oxychloride (20 ml) and diethylaniline (2 ml). The mixture was refluxed with stirring for 1 hour, cooled and poured onto ice (100 g). A yellow precipitate formed which was filtered, washed thoroughly with water and dried under vacuum. The crude product was recrystallised from methanol to yield a light-cream solid (1.2 g) which was dried to constant weight over phosphorus pentoxide.

### 3. 2-Benzylthio-N⁶-(2-isopentenyl)adenine

A mixture of 2-benzylthio-6-chloropurine (1.2 g) and isopentenylamine hydrochloride (1.04 g) in 1-butanol (25 ml) containing triethylamine (2.2 ml) was heated in a sealed tube at 110°C for 2 hours. The butanol was removed under vacuum and the mixture was shaken with ice-water (100 ml). The product was filtered recrystallised from ethanol with charcoal decolourisation and dried under vacuum with phosphorus pentoxide. Yield 0.64 g. Yield 1.3 g of 2-benzylthio-N⁶-(2-isopentenyl)adenine as a colourless solid. The crude product was used directly in the next step.

### 4. 2-Thio-N⁶-(2-isopentenyl)adenine

2-Benzylthio-N⁶-(2'-isopentenyl)adenine (0.64 g) was dissolved in liquid ammonia 62.5 ml) to yield a clear yellow solution. Sodium (approximately 200 mg) was added in small portions until a blue coloration persisted for 10 min. A small amount of solid ammonium chloride was added cautiously to remove excess sodium and the ammonia was allowed to evaporate to a small volume. Diethyl ether (65.5 ml) was added and the ether extract was extracted with water (62.5 ml). The aqueous extract was adjusted to between pH 4 and 5 with acetic acid when a creamy solid precipitated. After cooling, the product was filtered off and dried over phosphorus pentoxide under vacuum. Yield 340 mg.

Crude 2-thio-N⁶-(2-isopentenyl)adenine was converted to the potassium salt by suspension in water and addition of an equimolar amount of potassium hydroxide together with sufficient alcohol to effect solution. The solution was dried under vacuum and over phosphorus pentoxide.

### 5. N⁶-(2-isopentenyl)adenine 2-thioglucopyranuronide

2-Thio-N⁶-(2-isopentenyl)adenine, potassium salt (2.89 mmole) was dissolved in anhydrous methanol and MBTG (5 mmole) was added. The mixture was stirred for 24 hours at room temperature, during which time a creamy white solid was deposited. The reaction mixture was dried under vacuum and hydrolysed at room temperature by treatment with 5% aqueous sodium hydroxide (50 ml) to yield the free glucopyranuronide as the sodium salt. The mixture was neutralised by the careful addition of concentrated hydrochloric-acid with external cooling to yield a colourless precipitate of crude IP2SGN sodium salt.

The crude product was purified by reverse-phase chromatography recrystallised from ethanol and dried under vacuum over calcium chloride to yield a colourless, amorphous solid (150 mg). The product was sparingly soluble in 50% aqueous alcohol.

### Analysis

| UV | | |
|---|---|---|
| water, pH 1 | A max | 284, 241, 206 nm |
| water, pH 7 | | 278, 230 (shoulder) |
| water, pH 14 | | 283, 227 |

IP2SGN sodium salt showed the characteristic UV spectrum of 2-thio-substituted cytokinins and the spectrum was closely similar to that of authentic 2-methylthio N⁶-(2-isopentenyl)adenine. UV analysis confirmed the product as a 2-, N⁶-disubstituted adenine.

### HPLC

HPLC used a 15 x 0.46 cm octadecylsilica column eluted with a gradient of 0-60% methanol containing 0.2 M acetic acid over 30 min. at 1 ml/min. UV monitor at 270 nm.

IP2SGN sodium salt had a purity of 98+% and contained no detectable free 2-thio-N⁶-(2-isopentenyl)adenine (<0.1%).

### Hydrolysis by β-glucuronidase (GUS)

IP2SGN sodium salt (500 µg) in 500 µl of 50 mM sodium phosphate buffer, pH 7.0 was incubated with β-glucuronidase (GUS, Sigma Type G7896), 2500 Sigma "Fishman" units for 48 hours at 37°C. HPLC (conditions as above) showed partial removal (65%) of IP2SGN to produce a peak which co-chromatographed in a 1:1 mixture with 2-thio-N⁶-(2-isopentenyl)adenine. This test confirms the identity of IP2SGN sodium salt as a conjugate of 2-thio-N⁶⁻(2-isopentenyl)adenine and β-D-glucuronic acid, partially susceptible to GUS hydrolysis.

### EXAMPLE 1F

6-(3-methyl-but-2-enylamino)purine-8-yl-1-thio-β-D-glucopyranuronic acid, sodium salt
- Synonyms:: N⁶-(2'-isopentenyl)adenine-8-thioglucopyranuronic acid, sodium salt
N⁶-(2'-isopentenyl)adenine-8-thioglucuronide, sodium salt
- Abbreviation:: IP8SGN sodium salt

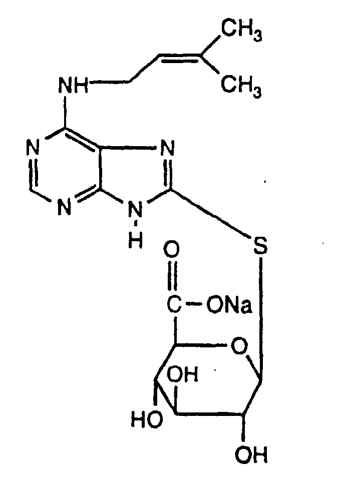

### Reaction 1

### 6-Hydroxy-8-thiopurine

4,5-Diamino-6-hydroxypyrimidine sulphate (25 g) and thiourea (100 g) were ground together and heated on an oil bath to 200°C for 30 min. The cooled, solidified product was dissolved in hot 1 M NaOH (500 ml), boiled with charcoal and filtered. The hot filtrate was acidified with cHCl and the hot solution was filtered to yield a red solid which was reprecipitated from hot basic solution, washed well with water and dried under vacuum at 80°C. Yield 5.28 g.

### Analysis

| UV | | |
|---|---|---|
| A max, pH 1 | 236, 292 nm | (lit. 234, 290 nm) |
| pH 11 | 234, 292 nm | (lit. 234, 290 nm) |

### Reaction 2

### 6-Hydroxy-8-benzylthiopurine

5.28 g 6-hydroxy-8-thiopurine was suspended in 78.5 ml of 1 M NaOH and diluted to 400 ml with water. 3.7 ml of benzyl chloride was added and the reaction mixture was stirred vigorously for 3 hours at room temperature, adjusted to pH 5 with glacial acetic acid and filtered. The product was washed thoroughly with water and dried overnight under vacuum at 80°C to give 7.33 g of a salmon-pink solid which was used without further purification.

### Reaction 3

### 6-Chloro-8-benzylthiopurine

6-Hydroxy-8-benzylthiopurine (7.33 g) was added to a mixture of phosphorus oxychloride (70 ml) and N,N-diethylaniline (7.5 ml), and the mixture refluxed for 2 hours to give a dark-red product. The mixture was concentrated under vacuum and the resulting syrup was poured slowly with stirring onto ice (400 g). The mixture was allowed to stand for 15 min., then made strongly alkaline with cold concentrated KOH. The mixture was triturated thoroughly to dissolve most of the syrup and acidified to pH 1 by the slow addition of cold concentrated HCI, with excess ice still present.

After standing for 1 hour at room temperature, the product was filtered off, washed thoroughly with water and dried under vacuum at 80°C for 2 hours to give 7.8 g of product.

### Reaction 4

### 8-Benzylthio-N⁶-(2'-isopentenyl)adenine

A mixture of 6-chloro-8-benzylthiopurine (3.9 g) and isopentenylamine hydrochloride (3.42 g) in 1-butanol (100 ml) containing triethylamine (7.5 ml) was refluxed for 2 hours. Most of the 1-butanol was removed under vacuum and the reaction mixture was cooled on ice and shaken with water (400 ml).

After refrigeration overnight, the mixture was filtered to yield a crude product which was purified by chromatography over 100 g silica gel eluted with a gradient of 0-2.5% MeOH in chloroform. The chromatographically pure product was recrystallised from methanol to yield 1.18 g of a colourless, amorphous solid.

### Analysis

### TLC (silica-2.5% methanol/chloroform)

Purity 98+%. No detectable impurities.

| UV | |
|---|---|
| 95% Ethanol/HCl | 307 nm |
| 95% Ethanol | 291 nm |
| 95% Ethanol/ammonia | 298 nm |

### Reaction 5

### 8-Thio-N⁶-(2'-isopentenyl)adenine

8-Benzylthio-N⁶-(2'-isopentenyl)adenine (1.18 g) was dissolve in liquid ammonia (125 ml) to yield a clear yellow solution. Sodium was added in small portions until a blue coloration persisted for 15 min. A small amount of solid ammonium chloride was added cautiously to remove excess sodium. The ammonia was evaporated to a small volume on a hot plate and ether (125 ml) was added.

After most of the remaining ammonia had been evolved the ether extract was extracted with water (2 x 65 ml). The aqueous extract (at pH 12-13) was cooled on ice and adjusted to pH 5 with glacial acetic acid. A creamy white solid precipitated which was filtered, washed thoroughly with water and dried overnight over calcium chloride to yield a virtually white, very light powder. Yield 760 mg.

Note: 8-Thio-N⁶-(2'-isopentenyl)adenine is readily oxidised in alkaline solution.

### Analysis

### HPLC (15 cm octadecylsilica, 0-80% methanol/0.2 M glacial acetic acid, 30 min., 1 ml/min., UV monitor at 300 nm)

Sharp, symmetrical peak with no detectable impurities. Purity 98+%.

| UV | |
|---|---|
| 95% Ethanol/HCl | 245, 241, 307 (sh), 315 nm |
| 95% Ethanol | 241, 305, 313 nm |

### Reaction 6

### IP8SGN sodium salt

8-Thio-N⁶-(2'-isopentenyl)adenine (600 mg) was suspended in 50 mM potassium hydroxide, (102 ml) containing 1% 2-mercaptoethanol as an antioxidant. Sufficient ethanol was added to dissolve the solid. The solution was dried under vacuum, over calcium chloride followed by phosphorus pentoxide. The dry product was dissolved in anhydrous methanol (100 ml) containing 2-mercaptoethanol (50µl) and MBTG (2 g) was added. The mixture was stirred for 24 hours at room temperature and dried under vacuum over calcium chloride and phosphorus pentoxide. The protected ester was hydrolysed for 1 hour at room temperature in 50 ml 5% sodium hydroxide.

After neutralization with concentrated hydrochloric acid the crude product was purified by reverse-phase and by normal-phase chromatography to yield a colourless solid which was dried to constant weight over calcium chloride. Yield 190 mg.

### Analysis of IP8SGN sodium salt

| UV | | |
|---|---|---|
| A max | Ethanol/pH 1 | 300 nm |
| | Ethanol/neutral | 285 (pronounced shoulder), 291, 301 nm |
| | Ethanol/pH 12 | 283 (shoulder), 290, 300 nm |

### TLC (silica-chloroform/methanol, 1/1)

IP8SGN sodium salt at loadings of 34, 68, 102 and 134 µg showed a single, sharp spot with no detectable contaminants. Purity 99.5+%. Content of free cytokinin base, 8-thio-N⁶-(2'-isopentenyl)adenine < 0.1%.

### HPLC (15 cm octadecylsilica, 0-80% methanol/0.2 M glacial acetic acid, 30 min., 1 ml, 300 nm)

Single, broad peak, tR 18.2 min. No contaminants detectable. Purity 99.5+%.

### GUS hydrolysis

IP8SGN sodium salt at 1 mg/ml was incubated with 2500 units GUS (Sigma) in 50 mM phosphate buffer, pH 7 at 37°C for 24 hours. TLC (1/1, methanol/chloroform) showed complete (> 95%) conversion of IP8SGN sodium salt to a compound which co-chromatographed with 8-thio-(2'-isopentenyl)adenine. IP8SGN sodium salt is therefore susceptible to GUS hydrolysis.

### EXAMPLE 1G

O-β-D-glucopyranuronosylzeatin, sodium salt
- Synonyms:: zeatin-O-β-D-glucopyranuronic acid, sodium salt,
zeatin-O-glucuronide, sodium salt
- Abbreviation:: ZOGN sodium salt

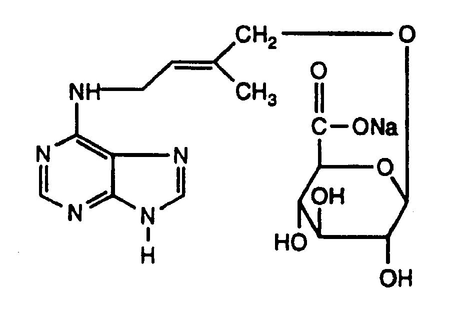

### Synthesis

### Trans-2-methyl-4-phthalimidobut-2-enyl-(2',3',4'-tri-O-acetyl-(3-D-glucopyranuronic acid methyl ester

Trans-1-hydroxy-2-methyl-4-phthalimido-but-2-ene, (1.87 g, (Corse & Kuhnle, 1972, Synthesis, pp. 618-619) and freshly-activated silver carbonate (9 g) were suspended in anhydrous ether (300 ml) containing molecular sieves (9 g). After stirring for 30 min. at room temperature MBTG (3.24 g) was added and the mixture was stirred in the dark for 2 days at room temperature. A further quantity of MBTG (1.62 g) was added and the mixture was stirred for a further 2 days. The reaction mixture was filtered, dried under vacuum to a colourless syrup which was further dried under vacuum over calcium chloride to yield a colourless, powderable foam. The crude product was freed from sugar impurities by chromatography over silica (200 g), eluting with chloroform. Yield of pure product was 2.45 g (55.4%).

### Trans-2-methyl-4-amino-but-2-enyl-β-D-glucopyranosyluronamide

Methyl trans-2-methyl-4-phthalimidobut-2-enyl-(2',3',4'-tri-O-acetyl-β-D-glucopyranuronate (2.45 g) was dissolved in anhydrous methanol (150 ml), cooled on ice, and ammonia was passed through the ice-cold solution for 6 hours. After removal of the methanol under vacuum the crude product was purified by chromatography over cellulose (200 g) and developed with butanol-ethanol-acetic acid-water (8:2:1:3 v/v). The eluate was dried under vacuum to yield a brown syrup which was used in the condensation step without further purification.

### Zeatin-O-β-D-glucuronic acid, sodium salt

The syrup from the previous step was heated in a sealed tube with 6-chloropurine (0.8 g) and triethylamine (1.5 ml) in methanol (25 ml) at 90°C for 4 hours to yield the amide. To convert the amide to the acid the methanol was removed under vacuum and 5% aqueous sodium hydroxide (25 ml) was added. After stirring at room temperature for 6 hours the mixture was carefully neutralised with 2 M hydrochloric acid and purified by reverse-phase chromatography over octadecylsilica (100 g), eluting with water. Crude ZOGN sodium salt containing unreacted 6-chloropurine, was dried under vacuum and purified by chromatography over silica (50 g) eluting with a 0-100% gradient of methanol in chloroform. After removal of the solvent under vacuum the product, as an amorphous solid, was dried under vacuum over calcium chloride. Yield 158 mg.

### Analysis

| UV | | |
|---|---|---|
| A max | aq. ethanol | 276 nm, 270 nm (shoulder), 283 nm (shoulder) |
| | aq. ethanol/HCl | 279 nm |
| | aq. ethanol/ammonia | 276 nm, 270 nm (shoulder), 283 nm (shoulder) |

### TLC (silica-1-butanol/glacial acetic acid/water, 12/3/5)

ZOGN sodium salt at 50 µg loading showed a single spot (Rf 0.16) with no detectable impurities. No zeatin (0.66 or 6-chloropurine (Rf 0.70) was detectable. Overall purity 98+%.

### HPLC (15 x 0.46 cm octadecylsilica column, UV monitor at 270 nm)

### Gradient elution (0-100% methanol over 30 min. at 1 ml/min.)

ZOGN sodium salt elutes as a fairly broad peak (tR 10.0 min.) with a small amount of inorganic impurity present with the solvent peak. Overall purity 97+%.

### Isocratic elution (50% aqueous methanol, 1 ml/min.)

Isocratic elution was used to measure zeatin content. ZOGN sodium salt had a retention time (tR) of 1.1 min., compared to the authentic trans zeatin tR of 2.2 min. HPLC of up to 96 µg of ZOGN sodium salt showed no detectable zeatin. The zeatin content was therefore < 0.1%. This was confirmed by silica gel TLC in chloroform/methanol 9/1. ZOGN sodium salt did not move from the origin in this system, but any contaminating zeatin was clearly separated at Rf 0.39. No zeatin was detected when 200 µg of ZOGN sodium salt was run. The detection limit for trans zeatin on TLC was 200 ng; therefore contamination with trans zeatin was confirmed as < 0.1%.

### Enzymatic hydrolysis

A sample of ZOGN sodium salt was incubated with β-glucuronidase (Sigma G9387) in 50 mM sodium phosphate buffer at pH 7.0 at 37°C. HPLC (50% isocratic methanol) showed virtually complete conversion to trans zeatin. The identity of trans zeatin in the enzyme hydrolysate was confirmed by co-chromatography of a 1:1 mixture of the hydrolysate and authentic trans zeatin in 3 different chromatographic systems.

### EXAMPLE 1 H

2-hydroxycinnamyl-β-D-glucopyranuronamide
- Synonyms:: o-coumaryl-β-D-glucopyranuronamide o-coumaryl glucuronamide
- Abbreviation:: CouGNamide

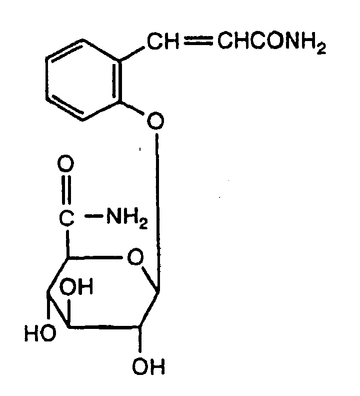

### Synthesis

Methyl o-coumarate (15 g) and MBTG (16.8 g) were ground together with quinoline (25 ml) to produce an homogeneous paste. Silver (I) oxide (10.7 g) was added in portions, with thorough mixing, while the mixture was cooled on ice. After addition was complete the reaction mixture was kept at room temperature for 3 h. The mixture was extracted with ether (1 I) and the ether extract washed with water (1 I). The ether extract was dried over anhydrous sodium sulphate and dried to a red syrup under vacuum. The crude syrup was extracted with petroleum ether (300 ml) to remove unreacted methyl o-coumarate then dried under vacuum to remove traces of petroleum ether. The crude product was recrystallised from ethanol with charcoal decolourisation to yield pure peracetylated methyl 2-hydroxycinnamyl-O-β-D-glucopyranuronate (3.5 g).

The peracetyl methyl ester (3.5 g) was dissolved in anhydrous methanol (250 ml) and hydrolysed by stirring at 0°C for 3 h with further anhydrous methanol (250 ml) which had been saturated with dry ammonia at -10°C. The ammonia and methanol were removed under vacuum and the crude product recrystallised from ethanol and dried under vacuum over calcium chloride to yield pure CouGNamide as a colourless, feathery solid (1.7 g).

### Analysis

### TLC (silica-chloroform/methanol, 1/1)

Single, sharp spot at Rf 0.63. No contaminants were detected at up to 50 µg loading, purity 98+%. Impurities, less than 0.5% of methyl o-coumarate; or coumarin.

### TLC (silica-chloroform/methanol/glacial acetic acid, 50/50/5)

Single, sharp spot at Rf 0.68. Purity 98+%. No detectable (< 0.5%) o-coumaric acid (Rf 0.80).

### EXAMPLE 1I

2-hydroxycinnamyl-β-D-glucopyranuronic acid
- Synonyms:: o-coumaryl-β-D-glucopyranuronic acid o-coumaryl glucuronide
- Abbreviation:: CouGN

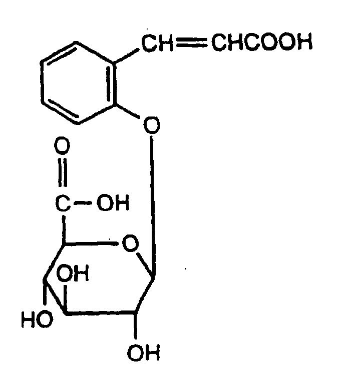

### Synthesis

Methyl o-coumarate (17.8 g) and MBTG (20 g) were ground together with quinoline (20 ml) to a uniform paste. Silver (I) oxide (13 g) was added in portions, with thorough mixing, while the mixture was cooled on ice. After addition was complete the reaction mixture was allowed to stand at room temperature for 3 h. The mixture was extracted with ether (1 I) and the ether extract washed with water (1 I). The ether extract was dried over anhydrous sodium sulphate and dried to a red syrup under vacuum. The crude syrup was extracted with petroleum ether (300 ml) to remove unreacted methyl o-coumarate then dried under vacuum to remove traces of petroleum ether. The crude product was recrystallised from ethanol with charcoal decolourisation to yield pure peracetylated methyl CouGN (2.9 g).

The peracetyl methyl ester (2.9 g) was suspended in methanol (250 ml) and 2 M sodium hydroxide (250 ml) added with stirring while the reaction mixture was cooled on ice. The mixture was stirred at room temperature for 1.5 h and adjusted to pH 2.5 with hydrochloric acid. The methanol was removed under vacuum and the crude product purified by XAD-2 chromatography. The chromatographic resin was first washed with water to remove inorganic salts and the product eluted with methanol. After drying under vacuum the product was recrystallised from ethanol and dried under vacuum over calcium chloride to yield pure CouGN as a colourless, amorphous solid (1.5 g).

CouGN is soluble at 5 mg/ml in aqueous buffer, pH 7, and in 50% aqueous alcohol to give a clear, colourless solution.

### Analysis

### TLC (silica-chloroform/methanol/glacial acetic acid, 50/50/1)

Major spot Rf 0.12 with a minor impurity at Rf 0.24. Purity 90+%. No detectable (< 1%) coumarin (Rf 0.90), methyl o-coumarate (Rf 0.91) or o-coumaric acid (Rf 0.80). The major product co-chromatographed with authentic CouGN prepared by the catalytic oxidation of 2-hydroxycinnamyl-O- -D-glucopyranoside.

### GUS hydrolysis

CouGN (5 mg) was dissolved in 50 mM sodium phosphate buffer, pH 7.0 (1 ml) and incubated with GUS (1000 units Sigma Type G5897) for 12 hours at 37°C. TLC showed 93.4% conversion to a compound which co-chromatographed with o-coumaric acid. O-coumaric acid is slowly converted (non-enzymatically) to coumarin over a period of a few days.

### EXAMPLE 2

### CYTOKININ GLUCURONIDES ARE HYDROLYZED BY β-GLUCURONIDASE, RELEASING UNMODIFIED CYTOKININS

An assay was developed to identify those cytokinin glucuronides which can be hydrolyzed by β-glucuronidase from E. coli. (Various features of the β-glucuronidase enzyme and gene are e.g. described in the following: Blanco & Nemoz, Biochimie 69, pp. 157-161, 1987; Jefferson et al., Proc. Natl. Acad. Sci. USA, Vol. 83, pp. 8447-8451, 1986; Levvy & Marsh, Advan. Carbohydrate Chem. 14, pp. 381-428; US 4,721,671).

The compound to be tested is incubated in 50 mM sodium phosphate buffer, pH 7.0, (generally 500 µg of the compound in 500 µl of the buffer) with β-glucuronidase (generally Sigma type G7896, 2500 Sigma "Fishman" units) for 12-24 h at 37°C. The presence of hydrolysis products is then determined using HPLC and TLC.

The table below shows the results of the above-described assay on a number of cytokinin-β-D-glucuronides and a cytokinin-β-D-glucuronamide.

**TABLE 1**

| Cytokinin-β-D-glucuronides (and a cytokinin-β-D-glucuronamide) as substrates β-glucuronidase from E. coli | |
|---|---|
| Hydrolysis | Compound |
| + | BA3GN sodium salt |
| - | BA3GNamide |
| - | BA9GN sodium salt |
| + | ZOGN sodium salt |
| + | IP3GN sodium salt |
| + | IP2SGN sodium salt |
| + | IP8SGN sodium salt |

Because the only substrates that have been used to assay the GUS enzyme from E. coli in transgenic organisms are O-glucuronides, and because it had been previously reported (Jefferson, "The GUS reporter gene system", Nature, Vol. 342, pp. 837-838, 1989) that the substrates of β-glucuronidase consist of D-glucuronic acid conjugated through a β-O-glycosidic linkage to an aglycone, it was surprising that certain N-glucuronides and S-glucuronides were also found to be good substrates for this enzyme. It is therefore also contemplated that such N- and S-glucuronide compounds may by used in assays for the β-glucuronidase enzyme from E. coli and plants, e.g. similar to the X-gluc assay referred to below.

In GB 2 197 653 A it is stated that by using the β-glucuronidase system and novel substrates, positive and negative selection using GUS activity may be possible. This hypothesis has, however, never been investigated, and enzymatic hydrolysis of such compounds has never been shown to be probable even on the basis of theoretical considerations regarding chemical characteristics of substrates for β-glucuronidase from E. coli. As shown here, not all glucuronides are substrates for the β-glucuronidase enzyme from E. coli, and it is not expected that most glucuronides are substrates for the GUS enzyme.

Responses in plant tissue expressing the β-glucuronidase gene from E. coli have been used as a method for evaluating whether certain glucuronides (including plant hormone precursors) can be hydrolyzed by the enzyme in vivo (Jefferson, "The GUS gene fusion system as a versatile tool for agricultural molecular biology", abstract from the International Congress on Genetic Manipulation in Plant Breeding held in Elsinore, Denmark, 11-16 September, 1988) Unfortunately, this approach is not feasible due to the occurrence of strong endogenous β-glucuronidase activity in plant tissue (see e.g. Example 3 below, as well as Hodal et al., "Detection, expression and specific elimination of endogenous β-glucuronidase activity in transgenic and non-transgenic plants", to be printed in Plant Science). The occurrence of this endogenous β-glucuronidase activity also means that by use of the procedure described by Jeffersen, it is impossible to determine whether the effects produced by the glucuronide actually are due to hydrolysis of the compound or whether they may be explained by the fact that the glucuronide itself is active. To be a suitable compound for selection purposes, a compound must be activated by the GUS enzyme and also without any significant activity in glucuronide form.

The assay described above can be used to screen for cytokinin glucuronides that are able to be hydrolyzed by a given β-glucuronidase enzyme, here exemplified by the β-glucuronidase enzyme from E. coli.

It was further determined using HPLC and TLC that unmodified active cytokinins are the product of the GUS hydrolysis (see the above examples relating to the preparation and analysis of novel cytokinin glucuronide compounds). Thus, it was e.g. found that hydrolysis of BA3GN sodium salt by β-glucuronidase from E. coli resulted in virtually complete (>99%) removal of the BA3GN sodium salt to yield a compound which co-chromatographed in a 1:1 mix with authentic BA. Similarly, incubation of ZOGN sodium salt with β-glucuronidase from E. coli was shown by HPLC (50% isocratic methanol) to give a virtually complete conversion to trans zeatin; and incubation of IP8SGN sodium salt with β-glucuronidase for 24 hours was shown by TLC (1:1, MeOH/chloroform) to give nearly complete (>95%) conversion of IP8SGN to a compound which co-chromatographs with 8-thio-(2-isopentenyl)adenine.

Similarly, other types of glucuronides may be screened for their ability to be hydrolyzed by a given β-glucuronidase. For example, incubation of o-coumaryl-β-D-glucuronide (5 mg in 1 ml buffer) for 12 h with β-glucuronidase (Sigma type G5897, 1000 units) was shown by TLC to give a 93.4% conversion to a compound which co-chromatographed with o-coumaric acid. Incubation of the other compounds listed in Table 1 (with the exception of the two compounds which did not act as substrates for β-glucuronidase from E. coli) gave similar results, i.e. gave hydrolysis products corresponding to those which were to be expected after hydrolysis by β-glucuronidase (see the above examples relating to the preparation of various glucuronide compounds). On the other hand, BA9GN, which as shown above in Table 1 is not a substrate for β-glucuronidase from E. coli, and which showed no detectable (<1%) production of BA after incubation for 18 h at 37°C with β-glucuronidase, does not induce shoot formation in tobacco leaf discs (Table 2 below).

### EXAMPLE 3

### INDUCTION OF SHOOT FORMATION FROM PLANT TISSUE WITH AND WITHOUT AN INTRODUCED β-GLUCURONIDASE GENE BY CYTOKININ GLUCURONIDES

Experiments were performed to determine whether cytokinin glucuronides (as well as a cytokinin glucuronamide) are able to induce shoot formation in vivo in plant material with and without, respectively, an introduced β-glucuronidase gene.

Seeds from a GUS-negative and a GUS-positive tobacco plant (Nicotiana tabacum 'Wisconsin 38') were germinated on MSO substrate (Murashige & Skoog substrate without hormones, described in Murashige & Skoog, Physiol. Plant. 15: 473-497, 1962, obtainable from Sigma, USA.). The GUS-positive plant material contained an introduced β-glucuronidase gene from E. coli (uidA) driven by the 35S promoter from cauliflower mosaic virus as described e.g. by Jefferson et al., The EMBO Journal, Vol. 6, No. 13, pp. 3901-3907, 1987. The original transgenic GUS-positive plants were produced using the traditional kanamycin based negative selection system as described e.g. by Burow et al., Plant Molecular Biology Reporter, Vol. 8(2), pp. 124-139, 1990. GUS-positive seedlings were identified using the X-gluc assay as described below in this example.

After 4-5 weeks the upper parts of the plants or shoots were transferred to a new MSO substrate and this procedure was repeated as necessary. In this way the plant material (both GUS-positive and GUS-negative) was maintained as sterile shoot cultures (see Burow et al., Plant Molecular Biology Reporter, Vol. 8(2), pp. 124-139, 1990). Discs were punched from the largest leaves (3-5 weeks old) and transferred to the substrates indicated below (Table 2). The basic substrate used was MSO, the various test compounds being added at several concentrations up to 250 µM. Small Petri dishes (∅=5 cm) containing about 6 ml of substrate were used, 3 leaf discs being placed on each Petri dish. Each treatment was repeated at least 4 times.

It was found that several cytokinin glucuronides induce shoot formation in plant tissues containing the β-glucuronidase enzyme. The results are shown in the following table:

**TABLE 2**

| Shoot formation induced by cytokinin-β-D-glucuronides (and a cytokinin glucuronamide) on leaf discs of tobacco with (GUS+) or without (GUS-) introduced β-glucuronidase genes | | |
|---|---|---|
| GUS- | GUS+ | Compound |
| - | - | None (control treatment) |
| + | + | BA (control treatment) |
| + | + | Zeatin (control treatment) |
| - | - | Glucuronic acid (control treatment) |
| + | + | BA3GN sodium salt |
| + | + | BA3GNamide |
| - | - | BA9GN sodium salt |
| + | + | ZOGN sodium salt |
| + | + | IP3GN sodium salt |
| + | + | IP2SGN sodium salt |
| + | + | IP8SGN sodium salt |
| "+" means shoot formation at concentrations below 250 µM | | |
| "-" means no shoot formation at concentrations below 250 µM (250 µM corresponds to approximately 100 mg/ml) | | |

It may be seen from the above table that all of the cytokinin glucuronides which induced shoot formation at a concentration of below 250 mM in leaf discs of tobacco containing an introduced β-glucuronidase gene also were able to induce shoot formation using a similar concentration in corresponding leaf discs which did not contain an introduced β-glucuronidase gene. On the other hand, the cytokinin glucuronide BA9GN, which was shown not to act as a substrate for β-glucuronidase from E. coli (see Example 2) did not result in shoot formation in leaf discs either with or without an introduced β-glucuronidase gene. However, the cytokinin glucuronamide BA3GNamide, which as shown in Example 2 did not act as a substrate for β-glucuronidase from E. coli, was found to induce shoot formation in both GUS-positive and GUS-negative leaf discs, indicating that cytokinin glucuronamides are converted to the corresponding cytokinin glucuronic acids in plant tissue. It thus appears that precursors for cytokinin glucuronides can function in vivo in the same manner as the cytokinin glucuronides themselves.

These results indicate that higher plants, in this case tobacco, possess endogenous β-glucuronidase activity. This finding is in accordance with that which has recently been reported by Hu et al. (Plant Cell Reports 9, pp. 1-5, 1990), who investigated the occurrence of GUS-like activity in 52 species of seed plants, and with the findings of Hodal et al. (to be printed in Plant Science). Hu et al. found that species expressing positive GUS-like activities are distributed in every key group of angiosperms as well as in some of the gymnosperms. (While Hu et al. did not detect G US activity in tobacco, this result may be explained by the fact that their assay was performed in vitro at pH 7. As shown below, the enzyme responsible for the native GUS activity in tobacco does not appear to be active at pH 7, but has been found to be active at pH 4-5).

These findings are in contrast to that which is disclosed in GB 2 197 653 A, which states that higher plants, including tobacco, contain no detectable β-glucuronidase activity. GB 2 197 653 A, which relates among other things to a method of monitoring expression of a gene of interest using the GUS gene, explains that the presence of GUS activity indicates the expression of the gene of interest and thereby implies that, since GUS activity is not found in higher plants, it is a relatively straightforward matter to monitor the expression of a gene of interest using the GUS gene. However, the above results show that this is not the case, and that the use of a GUS gene to monitor the presence of a gene of interest is not at all simple or straightforward, due to the fact that higher plants do in fact contain a significant intrinsic (background) β-glucuronidase activity.

In order to study the nature of the observed GUS activity in plant material with and without an introduced β-glucuronidase gene, the pH dependency of the GUS activity was determined using a standard histochemical GUS assay with "X-gluc" (5-bromo-4-chloro-3-indolyl-β-glucuronide).

The X-gluc assay may be carried out by first preparing an assay medium by dissolving 50 mg of X-gluc in a solution containing 25 ml 0.2 M NaPO₄ buffer, typically pH 7.0, 24 ml distilled water, 0.25 ml 0.1 M K₃(Fe(CN)₆), 0.25 ml 0.1 K₄(Fe(CN)₆) and 0.5 ml 1.0 M Na₂EDTA, followed by stirring until dissolved (about 30 min). Freshly cut or formaldehyde fixed sections (thickness less than 0.5 mm) or tissues at 37°C are then incubated in the X-gluc medium for from a few minutes to 24 hours. After incubation the sections are rinsed in sodium phosphate buffer or water and examined by microscope. GUS activity is seen as a blue staining of the treated plant material at the site of the enzyme activity (Jefferson, Plant Molecular Biology Reporter, Vol. 5, No. 4, pp. 387-405, 1987). For the purposes of the present invention assay times of 20 hours were typically used. After incubation the discs were treated with 96% ethanol to remove chlorophyll.

The results are shown in the following table:

**TABLE 3**

| pH-dependency of GUS activity in tobacco leaf discs with (+) and without (-) introduced GUS genes | | | | | | |
|---|---|---|---|---|---|---|
| pH | 3 | 4 | 5 | 6 | 7 | 8 |
| GUS(-) | 0 | + | + | 0 | 0 | 0 |
| GUS(+) | 0 | + | + | + | + | + |
| O: no reaction in X-gluc assay | | | | | | |
| +: blue reaction in X-gluc assay | | | | | | |

It may be seen that the enzyme responsible for the background β-glucuronidase activity in the tobacco leaf discs without an introduced β-glucuronidase gene is only active within a narrow pH range which corresponds to the internal pH of the plants (about pH 5), while the β-glucuronidase expressed by the introduced gene is active over a wide pH range of from 4 to 8. This pH dependency may explain why previous attempts to detect GUS activity in plants have been largely unsuccessful, leading to the mistaken conclusion (e.g. in GB 2197 653 A) that plants do not possess intrinsic GUS activity.

The fact that the β-glucuronidase activity shown above for the plant material without an introduced GUS gene in fact is the result of the hydrolysis of the cytokinin glucuronide substrate by β-glucuronidase, and not a result of a non-specific reaction which cleaves the substrate, e.g. a non-enzymatic acid hydrolysis of glucuronides (which are known to be cleaved at low pH values) was shown by testing the effect of inhibitors of various enzymes at pH 5 in non-genetically transformed plant material (i.e. plant material having only native GUS activity). Testing was performed using the X-gluc assay described above at pH 5.0 for 20 hours.

**TABLE 4**

| Test of inhibitors at pH = 5.0. Non-transformed material | | | | | |
|---|---|---|---|---|---|
| | Concentration (mM) | | | | |
| | 0 | 0.1 | 1 | 10 | 50 |
| Saccharo 1,4-lactone | + | + | 0 | 0 | 0 |
| Gluconolactone | + | + | + | + | + |
| UDP-glucuronide | + | + | + | + | + |
| Glucuronic acid | + | + | + | 0 | 0 |
| Galactose | + | + | + | + | + |
| Methylumbellipheryl glucuronide | + | + | + | + | 0 |
| EDTA | + | + | + | + | + |
| 0 = no reaction, i.e. completely white leaf disc | | | | | |
| += blue staining with X-gluc | | | | | |

The six inhibitors tested have the following effects:

Saccharo 1,4-lactone is an inhibitor which is specific for β-glucuronidase enzymes (in other words, it inhibits only β-glucuronidases and it inhibits all β-glucuronidases). It is generally accepted that GUS activity which can be inhibited by this compound results from the action of a β-glucuronidase enzyme.

Gluconolactone is an inhibitor of β-glucosidases. It corresponds to saccharo 1,4-lactone, with the exception that it is specific for β-glucosidases.

UDP-glucuronide is a substrate for UDP-glucuronide transferases.

Glucuronic acid is the product of every β-glucuronidase reaction and therefore a product inhibitor of β-glucuronidases and other glucuronic acid forming enzymes.

Galactose is an inhibitor of UDP-glucuronide-transferase dependent reactions.

Methylumbellipheryl glucuronide is a substrate for all β-glucuronidases thus far investigated and is thus a competitive inhibitor of GUS enzymes.

Since it was also found that EDTA (which inhibits UDP-glucuronide transferases) has no effect, it is unlikely that such transferases are involved in the observed GUS activity. The above table shows that those compounds which should inhibit a transferase enzyme have no effect even at very high concentrations. It may furthermore be seen that gluconolactone has no inhibiting effect, and it is therefore unlikely that the GUS activity is related to a β-glucosidase activity.

It may further be seen that the GUS specific inhibitor saccharo 1,4-lactone is a strong inhibitor, that glucuronic acid (product inhibition of β-glucuronidase) is a medium strength inhibitor and that methylumbellipheryl glucuronide (a GUS substrate and therefore a competitive substrate to X-gluc if a β-glucuronidase enzyme is responsible for the hydrolysis of X-gluc) is a weak inhibitor. The GUS activity in tobacco therefore fulfills all the necessary criteria to be classified as resulting from a β-glucuronidase enzyme. It may therefore be concluded that the plants contain a β-glucuronidase enzyme.

A corresponding series of experiments was performed in order to ascertain whether the effect of the inhibitors was sufficiently fast to be able to inhibit the enzyme. In this series, the plant material was pre-incubated in the test compounds (inhibitors) for 24 hours before the X-gluc assay was performed with the same test compounds. The results obtained were identical to those obtained without preincubation, which indicates that the inhibitors penetrate into the plant tissue fast enough to inhibit the X-gluc assay before blue staining can occur.

Results similar to those described above were obtained in another investigation of the occurrence of GUS activity in plants. In this case, the pH dependency of the histological GUS reaction was tested in a number of plant species at pH values between 3 and 8 both without and with the GUS-specific inhibitor saccharo 1,4-lactone.

The assay used was the X-gluc assay described above. The assay was carried out at 37°C for 20 hours. Leaves were dissected (with sterile razor blades) so that each leaf was tested at a number of different pH values.

As shown by the table below, this investigation confirmed that plants do indeed possess native GUS activity, that this activity is the result of an enzymatic reaction (no reaction in the presence of the GUS-specific inhibitor) and that the enzyme is primarily active at pH values of about 4-5.

**TABLE 5**

| Histological GUS reaction at different pH values | | | | | | | |
|---|---|---|---|---|---|---|---|
| pH during assay | 3 | 4 | 5 | 6 | 7 | 8 | 3-8 |
| Saccharo 1,4-lactone (mM) | 0 | 0 | 0 | 0 | 0 | 0 | 10 |
| Plant species | | | | | | | |
| Sugar beet, wild type | + | + | + | + | (+) | 0 | 0 |
| Sugar beet, transgenic 1) | + | + | + | + | + | + | (+) |
| Sugar beet, transgenic 2) | + | + | + | + | (+) | 0 | 0 |
| Wheat | 0 | + | + | + | 0 | 0 | 0 |
| Wheat, albino | 0 | + | + | + | 0 | 0 | 0 |
| Oilseed rape | + | + | + | (+) | (+) | 0 | 0 |
| Tobacco | 0 | + | + | 0 | 0 | 0 | 0 |
| Tobacco, transgenic 1) | 0 | + | + | + | + | + | 0 |
| Tobacco, transgenic 2) | 0 | + | + | 0 | 0 | 0 | 0 |
| Sitka spruce 4) | 0 | + | + | 0 | 0 | 0 | 0 |
| Rhubarb 3) | n | n | + | + | + | + | 0 |
| Pea 3) | 0 | + | + | 0 | 0 | 0 | 0 |
| Oxalis 3) | n | n | n | n | (+) | n | 0 |
| Chenopodium quinoa pollen | n | n | n | 0 | + | n | n |

| | | | | | | | |
|---|---|---|---|---|---|---|---|
| 1) Leaf tissue expressing the GUS gene from E. coli 2) Transgenic leaf tissue only containing a kanamycin resistance gene (NPT), without an introduced GUS gene 3) Stem or petiole tissue 4) Embryogenic callus tissue + Blue reaction (+) Low frequency of GUS activity (faint blue reaction) 0 No reaction n Not determined | | | | | | | |

The fact that plants have a general intrinsic GUS activity makes it possible to use a gene encoding glucuronide permease as a positive selection gene without the use of any other selection gene by taking advantage of a increased uptake of a glucuronide compound by transformed cells. It has for example been found that glucuronides are not easily taken up into plant cells through plasma membranes. If a glucuronide permease gene is introduced into a cell, however, glucuronides will more readily be able to cross the plasma membrane and enter the cell. The glucuronides will then be available for cleavage by the intrinsic GUS enzyme in the transformed cells. In contrast, the glucuronide in question will not be available for non-transformed cells, whereby a positive selection effect will be achieved. Similarly, positive selection may also be performed using other permeases and other types of compounds which either are activated in the transformed cells by an intrinsic enzyme or which otherwise exert a biological effect in the transformed cells into which they are transported.

### EXAMPLE 4

### CYTOKININ GLUCURONIDES ARE STABLE AND INACTIVE

The effect of the cytokinin glucuronide BA3GN sodium salt, is blocked when the GUS activity in the plant tissue containing this cytokinin glucuronide is inhibited. In other words, the cytokinin glucuronide is inactive in itself. Furthermore, the cytokinin glucuronide has been shown to be stable in the growth medium as well as in plant tissue when β-glucuronidase is not present, as shown by the fact that the specific GUS inhibitor, saccharo 1,4-lactone (SL), strongly inhibits shoot formation induced by the cytokinin glucuronide BA3GN sodium salt, but only weakly inhibits shoot formation induced by the free cytokinin BA (using the basic method described above in Example 3):

**TABLE 6**

| Inhibition by saccharo 1,4-lactone (SL) of shoot formation induced by BA (0.5 mg/l) and BA3GN sodium salt (15 mg/l) | | | | |
|---|---|---|---|---|
| Treatment | Number and relative number of regenerated shoots per leaf disc | | | |
| SL (mM) | BA | | BA3GN sodium salt | |
| 0 | 4.3 | 100% | 5.6 | 100% |
| 16 | 3.6 | 84% | 1.4 | 25% |

By comparing the results in the above table with those given above in Table 2, it may be seen that BA3GN sodium salt, which induces shoot formation in both GUS-positive and GUS-negative tobacco leaf discs, does not induce shoot formation in corresponding leaf discs in the presence of the β-glucuronidase specific inhibitor saccharo 1,4-lactone.

Saccharo 1,4-lactone (SL) is not a stable compound at the pH used in this example. An equilibrium exists between SL and saccharic acid (SA). This equilibrium is reached only slowly, and the conversion of SL to SA is followed by a drop in pH. The pH must therefore be adjusted several times during a one week period prior to use of SL until the pH has stabilized in the SL-containing substrate.

The fact that cytokinin glucuronides are stable and inactive when not in the presence of β-glucuronidase is further shown by the fact that BA9GN, which cannot be hydrolyzed by β-glucuronidase, cannot induce shoot formation in plant material containing β-glucuronidase activity (see Examples 2 and 3).

It is important that the cytokinin glucuronide compounds are inactive and stable in substrates not containing a β-glucuronidase enzyme, since this stability is a prerequisite for the proper functioning of the positive selection system which uses them.

Not all glucuronic acid derivatives can be expected to be stabile, however. For example, ester glucuronides will not be stable in plant tissue which contains non-specific esterases. This means that the cytokinin glucuronide compounds prepared and used according to the present invention (β-D-glucuronides coupled to the aglycon via glycosidic O, S and N atoms) fulfill the prerequisite for stability. On the other hand, compounds having other types of glucuronide linkages, e.g. amide or ester β-D-glucuronides, are not expected to be selectively hydrolyzed by β-glucuronidases, due to the occurrence of non-specific esterases and amidases in plants.

Referring to Example 3 above, it has thus been shown that the tested cytokinin glucuronides, which as shown in this example do not in themselves possess cytokinin activity, are cleaved in vivo by the action of β-glucuronidase - either in the form of endogenous background β-glucuronidase or as a result of an introduced β-glucuronidase gene - whereby free cytokinin is released.

### EXAMPLE 5

### INDUCTION OF SHOOT FORMATION FROM PLANT TISSUE CONTAINING AN INTRODUCED β-GLUCURONIDASE GENE USING STEROL-GLUCURONIDES

Other glucuronides, including glucuronides of sterols, glycyrrhizic acid and hydroxyquinoline, have been shown to by hydrolyzed by β-glucuronidase and to result in shoot formation on modified substrates in which the hydrolysis products are essential for shoot formation. This is shown in Examples 5 and 6.

Experiments were performed to determine the shoot inducing effect of two sterol-glucuronides, β-sitosteryl-β-D-glucuronide (SG) and cholesteryl-β-D-glucuronide (CG), when sterol synthesis is inhibited in the tissues. The basic methods employed correspond essentially to those described for Example 3 above. However, in addition to one or both of the above-mentioned sterol-glucuronides, the substrate contained 0.1 mM tridemorph and 5 mg/l BA3GN sodium salt. The number of shoots were registered after 30 days.

Tridemorph (4-tridecyl-2,6-dimethyl morpholine) is a fungicide which inhibits the synthesis of sterols and similar compounds. Tridemorph has an inhibiting effect on shoot regeneration (although not a fatal effect on the explants) when the plant tissue is not supplied with sterols. Thus, in the absence of free sterols, shoot formation should effectively be prevented.

CG was obtained from Sigma, USA, and SG was synthesized according to the procedure described by Ando et al. in J. Antibiotics 73, p. 408, 1970.

The results obtained are shown in the following table:

**TABLE 7**

| Regenerated shoots per tobacco leaf disc on substrates supplied with tridemorph (0.1 mM) and BA3G N sodium salt (5 mM) | | | | |
|---|---|---|---|---|
| Compound | Concentration mg/l | | | |
| Sitosteryl-β-D-glucuronide | 0 | 0 | 12.5 | 12.5 |
| Cholesteryl-β-D-glucuronide | 0 | 50 | 0 | 50 |
| Shoots per leaf disc | 0.3 | 0.3 | 2.8 | 4.0 |

The above table shows that sitosteryl-β-D-glucuronide is able to counteract the shoot inhibiting effect of tridemorph and that the combination of sitosteryl-β-D-glucuronide and cholesteryl-β-D-glucuronide provides the greatest shoot formation. Thus, positive selection according to the invention using an introduced β-glucuronidase gene is possible using e.g. one or both of the above sterol-glucuronide compounds together with a shoot inhibiting compound such as tridemorph. These results indicate that also other sterols and sterol-like compounds can be used for positive selection from tissue containing an introduced β-glucuronidase gene.

An advantage of using these very slightly soluble compounds for positive selection is that their effect will presumably be very local, since the compounds do not diffuse from cell to cell when the hydrophilic glucuronide moiety is cleaved off by a β-glucuronidase enzyme. In other words, these compounds can be used to prevent cross feeding during the selection procedure.

This experiment further indicates that the shoot inhibiting effect of tridemorph, and thus also other compounds which inhibit sterol synthesis, can be counteracted by adding sterols and sterol derivatives to the substrate, whereby a selection system based on providing transgenic cells with sterols and sterol-like compounds can be established with the above-mentioned advantages.

### EXAMPLE 6

### INDUCTION OF SHOOT FORMATION FROM PLANT TISSUE WITH AND WITHOUT AN INTRODUCED β-GLUCURONIDASE GENE USING SITOSTERYL-β-D-GLUCURONIDE

An experiment similar to that described in Example 5 was performed on both transformed and non-transformed tobacco leaf discs using sitosteryl-β-D-glucuronide and either BA or BA3GN sodium salt.

The methods employed were essentially those described above in Example 5. In this experiment, tridemorph was added to the substrate at a concentration of 0.1 mM and sitosteryl-β-D-glucuronide was added at a concentration of 121 mg/l. The substrate contained in addition either 1.88 mg/l BA3GN sodium salt or 0.1 mg/l BA. The number of shoots was registered after 40 days.

The number of shoots obtained was as follows:

| Regenerated shoots per leaf disc | | | | |
|---|---|---|---|---|
| | BA | | BA3GN sodium salt | |
| Compound | GUS+ | GUS- | GUS+ | GUS- |
| Sitosteryl-β-D-glucuronide | 2.4 | 1.9 | 2.4 | 0.1 |

It may be seen that, as was the case in Example 5 above, the presence of sitosteryl-β-D-glucuronide was able to counteract the shoot inhibiting effect of tridemorph. Furthermore, when sitosteryl-β-D-glucuronide and tridemorph were used together with BA3GN sodium salt, selective shoot formation was obtained in the GUS-positive leaf discs, while the GUS-negative leaf discs on the substrate containing BA3GN sodium salt had virtually no shoot formation.

These results also indicate that the use of a combination of different glucuronides (here BA3GN and SG instead of BA and SG) may improve the selective response from the transgenic tissues.

Since sterols and steroids are also important growth regulators in animal cells, corresponding selection procedures may also be used for the selection of animal cells which express β-glucuronidase.

### EXAMPLE 7

### DEARMED AGROBACTERIUM STRAINS PRODUCE CYTOKININS

It has been found that certain Agrobacterium strains induce shoot formation due to production of shoot-inducing substances during co-cultivation. Such strains should normally be avoided when GUS hydrolysis of cytokinin glucuronides is to be employed for the purposes of selection of genetically transformed cells, since these strains alone may induce shoot formation and thereby interfere with the selection process.

As an example, the table below shows the results of an experiment with two different Agrobacterium strains, one of which induces shoot formation on tobacco leaf discs after co-cultivation.

The methods used correspond essentially to those described in Example 13, but after co-cultivation, the leaf discs were transferred to MSO substrate without hormones containing 300 mg/l cefotaxime and 300 mg/l carbenicillin.

The number of regenerated shoots was registered 4 weeks after co-cultivation.

**TABLE 8**

| Induction of shoot formation on tobacco leaf discs on a hormone-free substrate after co-cultivation with dearmed Agrobacterium | | | |
|---|---|---|---|
| Agrobacterium strain | Plasmid | Genes in T-DNA | Number of shoots per leaf disc |
| 1. C58X | T37 | GUS and NPT | 9.3 |
| 2. LBA4404X | PAL4404 | GUS and NPT | 0 |
| 3. C58Y | T37 | None | 4.8 |
| 4. LBA4404Y | PAL4404 | None | 0 |
| 5. None | - | - | 0 |

It is seen that the shoot inducing properties of some of the Agrobacterium strains are not dependent on the genes contained in the T-DNA. This means that genes outside the T-DNA region are responsible for the shoot induction.

A gene outside the T-DNA region responsible for the cytokinin production has been named tzs (Morris, R.O. Ann. Rev. Plant Physiol. 37, pp. 509-538, 1986). Strain C58 contains the tzs gene, a non-transferable gene which codes for the synthesis of zeatin during co-cultivation. Strain LBA4404 does not contain the tzs gene. The fact that the shoot-inducing strains contain a plasmid containing the tzs gene indicates that this gene may be responsible for the shoot-inducing properties observed in these investigations and that strains of Agrobacterium containing the tzs gene should be avoided in cytokinin-based selection systems. Any other Agrobacterium strains that induce shoot formation should normally also be avoided.

### EXAMPLE 8

### POSITIVE SELECTION OF TRANSGENIC SHOOTS USING THE CYTOKININ GLUCURONIDE BA3GN SODIUM SALT

Genetically transformed shoots may be selected using cytokinin glucuronides.

A series of experiments was performed to test the effectiveness of the positive selection system using the cytokinin glucuronide BA3GN sodium salt in concentrations of 7.5 and 15 mg/l. The experiments were performed on wild type tobacco leaf discs using 2 different Agrobacterium strains as well as various co-cultivation substrates. The transformation method used was that described below in Example 13, with the exception that Gamborg B5 substrate (Gamborg et al., Exp. Cell Res. 50: 151-158, 1968; obtainable from Sigma, USA) was used instead of MSO. The results given below are averages based on two independent experiments, each of which was carried out on 27 leaf discs per treatment.

**TABLE 9**

| Positive selection of genetically transformed shoots using the cytokinin glucuronide BA3GN sodium salt | | | |
|---|---|---|---|
| 7.5 mg/l BA3GN sodium salt | | | |
| Agrobacterium strain | Co-cultivation substrate | GUS+ shoots per leaf disc | % GUS+ shoots among total shoots |
| BS10 | B5 | 0.1 | 6.6 |
| BS10 | B5 + ammon. | 0.02 | 0.7 |
| BS10 | B5 + ammon. + SL | 0.3 | 6.1 |
| BS10 | average | 0.1 | 4.4 |
| | | | |
| LDH1 | B5 | 0.2 | 7.8 |
| LDH1 | B5 + ammon. | 0.2 | 5.3 |
| LDH1 | B5 + ammon. + SL | 0.2 | 4.7 |
| LDH1 | average | 0.2 | 5.9 |
| Both | average | 0.2 | 5.1 |

| 15 mg/l BA3GN sodium salt | | | |
|---|---|---|---|
| Agrobacterium strain | Co-cultivation substrate | GUS+ shoots per leaf disc | % GUS+shoots among total shoots |
| BS10 | B5 | 0.3 | 6.0 |
| BS10 | B5+ammon. | 0.4 | 8.7 |
| BS10 | B5+ammon.+SL | 0.3 | 5.5 |
| BS10 | average | 0.3 | 6.7 |
| | | | |
| LDH1 | B5 | 0.4 | 9.0 |
| LDH1 | B5 + ammon. | 0.3 | 7.6 |
| LDH1 | B5 + ammon. + SL | 0.3 | 7.7 |
| LDH1 | average | 0.3 | 8.1 |
| Both | average | 0.3 | 7.4 |
| B5: Gamborg B5 substrate, pH 5.4 | | | |
| Ammon.: Co-cultivation substrate with 75 mM ammonium nitrate | | | |
| SL: Co-cultivation substrate with 25 mM saccharic acid + 25 mM saccharo 1,4-lactone from a stabilized solution | | | |
| GUS+: Shoots expressing GUS activity when assayed at pH 7 as described in Example 3 | | | |

Strain BS10 introduces a GUS gene driven by a modified 35S promotor not active in Agrobacterium, as described by Janssen & Gardner in Plant Molecular Biology, 14, pp. 61-72, 1989. Strain LDH1 introduces a GUS gene driven by the unmodified 35S promotor.

The results in Table 9 show that positive selection of transgenic shoots expressing an introduced β-glucuronidase gene is possible using BA3GN sodium salt. These results are very significant, and were unexpected, since it was found as described in Example 3 that cytokinin glucuronides were able to induce shoot formation in leaf discs not containing an introduced β-glucuronidase gene. The different treatments during co-cultivation do not appear to have any significant effect on selection.

The results shown above indicate also that the use of an Agrobacterium strain with an active β-glucuronidase gene (strain LDH1 expresses GUS activity in bacteria) does not affect the transformation system compared to a strain which does not have an active β-glucuronidase gene (strain BS10 does not express GUS activity in bacteria).

### EXAMPLE 9

### SELECTION OF GENETICALLY TRANSFORMED SHOOTS USING THE CYTOKININ GLUCURONIDE ZEATIN-O-β-GLUCURONIC ACID

Using essentially the same procedure as described in Example 13 transgenic tobacco shoots were prepared and selected using the cytokinin glucuronide zeatin-o-β-glucuronic acid (ZOGN) as the positive selection agent.

Co-cultivation was carried out for 3 days, the inoculum density corresponding to an OD of 1.5 at 660 nm. The substrate used after co-cultivation was MSO containing 300 mg/l carbenicillin, 300 mg/l cefotaxime and 0.1 mg/l indole acetic acid (IAA). Subcultivation after 3 weeks was to the same substrate but without IAA. The pH in all substrates used in this example was 8.0. 18 leaf discs were used for each treatment.

Five weeks after co-cultivation the shoots were transferred to an MSO substrate containing 200 mg/l kanamycin sulfate, 32 mM saccharic acid, 300 mg/l cefotaxime and 300 mg/l carbenicillin, pH 8.0. Saccharic acid, an inhibitor of β-glucuronidase enzymes, was added to stop further conversion of zeatin glucuronide to zeatin. Together with the β-glucuronidase gene an NPT gene providing resistance to kanamycin was co-transferred. Non-transformed shoots (negative controls) survived, but the growth of these shoots was retarded on this substrate, while all of the transformed shoots (positive controls) containing an active NPT gene survived without any growth retardation.

The number of shoots was registered and the number of GUS-positive shoots among the total number of shoots was determined by the X-gluc assay (Example 3) 3 weeks after the last subcultivation (8 weeks after co-cultivation). The results are shown below:

**TABLE 10**

| Positive selection of genetically transformed shoots using ZOGN sodium salt | | |
|---|---|---|
| ZOGN mg/l | GUS-positive shoots per leaf disc | % GUS-positive shoots among total shoots |
| 0.07 | 0.3 | 18.5 |
| 1.0 | 2.6 | 40.3 |
| 15.0 | 0.2 | 5.1 |
| 0.07+SL* | 0 | 0 |

| | | |
|---|---|---|
| * SL: 2 mM saccharo 1,4-lactone | | |

The results given in the above table show that a successful positive selection of genetically transformed shoots using ZOGN was achieved. The induction of transgenic shoots was inhibited when the β-glucuronidase specific inhibitor saccharo 1,4-lactone was added to the substrate, which shows that the growth of transgenic shoots and thus the success of the positive selection was dependent upon the β-glucuronidase catalyzed conversion of ZOGN to zeatin.

### EXAMPLE 10

### SELECTION OF GENETICALLY TRANSFORMED SHOOTS USING ZEATIN-O-β-GLUCURONIC ACID AT VARIOUS TEMPERATURES

The temperature dependency of the positive selection system using cytokinin glucuronides was investigated using zeatin-o-β-glucuronic acid (ZOGN) as the positive selection agent at 25°C, 30°C and 35°C.

Transgenic tobacco shoots were prepared using essentially the same procedure as described below in Example 13. Co-cultivation was carried out for 3 days, the inoculum density corresponding to an OD of 1.5 at 660 nm. The substrate used after co-cultivation was MSO containing 10 mg/l 2-(2-hydroxyethylamino)-6-benzylamino-9-methylpurine (9-met), 350 mg/l carbenicillin, 350 mg/l cefotaxime and 0.1 mg/l indole acetic acid (IAA) and ZOGN sodium salt as indicated. 9-met (obtained from Apex Organics Ltd., UK) was added to inhibit glycosylation of zeatin and zeatin derivatives. 18 leaf discs were used for each treatment.

Seven weeks after co-cultivation the shoots were transferred to an MSO substrate containing 300 mg/l kanamycin sulfate, 350 mg/l cefotaxime, 350 mg/l carbenicillin, 0.1 mg/l IAA and 10 mg/l 6-(m-hydroxybenzylamino)-purine (OH-BA) and placed at a temperature of 25°C. OH-BA can be prepared as described by Kaminek et al. (Plant Growth Reg. 6, pp. 113-120, 1987).

After six weeks on the kanamycin-containing substrate the number of green shoots was registered. Resistance to kanamycin indicates that the shoot is transgenic, because together with the β-glucuronidase gene an NPT gene providing kanamycin resistance was co-transferred. No non-transformed shoots (negative controls) survived on this substrate, while all of the transformed shoots (positive controls) containing an active NPT gene survived.

The percentage of kanamycin resistant shoots among the total number of regenerated shoots was calculated as the number of green shoots surviving on the kanamycin-containing substrate divided by the number of shoots transferred to the kanamycin-containing substrate. The results are shown in the table below:

**TABLE 11**

| Positive selection of genetically transformed shoots using ZOGN sodium salt at different temperatures | | | |
|---|---|---|---|
| T °C | ZOGN mg/l | Kanamycin-res. shoots per leaf disc | % Kanamycin-res. shoots among total shoots |
| 25 | 1 | 0.3 | 4.3 |
| 30 | 1 | 1.3 | 14.8 |
| 35 | 1 | 0.2 | 26.7 |
| | | | |
| 25 | 15 | 0 | 0 |
| 30 | 15 | 2.4 | 26.2 |
| 35 | 15 | 0.2 | 18.2 |

The bioassay described in this example was performed on a linked co-transferred gene. This means that the β-glucuronidase gene is used for selection, while the resistance resulting from the introduced co-transferred NPT gene (kanamycin resistance gene) is assayed.

The results above show that, in addition to the β-glucuronidase gene, a co-transferred gene is also expressed when selection is performed using the β-glucuronidase gene. In addition, it can be seen that selection at elevated temperatures (i.e. about 30-35°C) improves the selection of shoots per leaf disc and also the fraction of transgenic shoots among the total number of regenerated shoots.

By performing the X-gluc assay at different temperatures, it has been observed in connection with the present invention that the intrinsic β-glucuronidase activity occurring in plants is inhibited at high temperatures, while the introduced β-glucuronidase activity is not affected. It was thus found that the intrinsic β-glucuronidase activity gradually decreased with increasing temperatures up to about 60°C, at which temperature there was essentially no intrinsic β-glucuronidase activity (as determined by the X-gluc assay). This may explain the improvement of the selection procedure at elevated temperatures.

### EXAMPLE 11

### POSITIVE SELECTION COMPARED TO AND COMBINED WITH NEGATIVE SELECTION

It has been shown that the positive selection system described herein is very efficient and advantageous compared to traditional kanamycin-based negative selection. However, good results are also obtained when the positive selection system is employed together with traditional negative selection.

The table below thus shows the results, in terms of the number of GUS-positive tobacco shoots per leaf disc and the percentage of GUS-positive shoots among the total number of shoots, for positive selection using BA3GN sodium salt, traditional negative selection using kanamycin and BA, as well as positive selection and negative selection in combination. In addition, the experiment included selection using BA3GN sodium salt together with saccharo 1,4-lactone (SL) (a strong specific inhibitor of the introduced β-glucuronidase).

The methods used were essentially as described below in Example 13, but Gamborg B5 substrate was used instead of MSO substrate.

**TABLE 12**

| Positive selection combined with negative selection | | | | | | |
|---|---|---|---|---|---|---|
| Selection substrate Conc.** | | Kanamycin conc.** | GUS+ shoots per leaf disc | | GUS+ shoots among total shoots | |
| | | | No. | Compared to BA + kanamycin | % | Compared to BA + kanamycin |
| BA | 1 | 300 | 0.01 | 1x | 4.3 | 1x |
| BA3GN* | 15 | 300 | 0.1 | 10x | 47.1 | 11x |
| BA3GN* | 15 | 100 | 0.2 | 20x | 3.2 | 0.7x |
| BA3GN* | 15 | 33 | 0.4 | 40x | 4.7 | 1.1x |
| BA3GN* | 15 | 0 | 0.3 | 30x | 7.4 | 1.7x |
| BA3GN* | 15 | 0 | | | | |
| + SL (10 mM) | | | 0.02 | 2x | 0.9 | 0.2x |

| | | | | | | |
|---|---|---|---|---|---|---|
| * sodium salt ** Concentrations in mg/l | | | | | | |

The experiment with BA together with kanamycin, which is the traditional negative selection system, was repeated as two independent experiments with 54 leaf discs per experiment. A single G US-positive shoot was detected among a total of 23 selected shoots. The results for BA3GN sodium salt were obtained using 324 leaf discs. The experiment with SL (saccharo 1,4-lactone) was performed, once with a total of 162 leaf discs.

The above table shows that positive selection using 15 mg/l BA3GN sodium salt (without kanamycin) gave 30 times as many GUS-positive shoots per leaf disc as the traditional negative selection system using 1 mg/l BA and 300 mg/l kanamycin sulfate. Furthermore, a greater percentage of the total number of shoots were GUS-positive when positive selection was used (7.4%) than when negative selection was used (4.3%).

Advantageous results were also obtained using a combination of positive and negative selection, i.e. substituting a cytokinin in the traditional kanamycin-based negative selection system with a cytokinin glucuronide (BA3GN sodium salt). Thus, when 15 mg/l BA3GN sodium salt was combined with 300 mg/l kanamycin sulfate, the percentage of GUS-positive shoots was 11 times that obtained using BA and kanamycin, and when 15 mg/l BA3GN sodium salt was combined with 33 mg/l kanamycin sulfate, the number of GUS-positive shoots per leaf disc was 40 times that obtained using BA and kanamycin.

When 15 mg/l BA3GN sodium salt was combined with 10 mM of the GUS inhibitor SL, both the number of GUS-positive shoots per leaf disc and the percentage of GUS-positive shoots was drastically reduced compared to when 15 mg/l BA3GN sodium salt was used alone. This shows that the introduced GUS gene is responsible for the advantageous results obtained using the positive selection system, since the addition of SL to the growth medium severely inhibits the β-glucuronidase catalyzed conversion of inactive cytokinin glucuronide (BA3GN sodium salt) to active cytokinin in cells grown on this substrate, thereby leading to the observed reduction in the number of shoots induced. The positive selection system thus functions as intended, i.e. using an introduced β-glucuronidase to cleave a cytokinin glucuronide in genetically transformed cells, thereby releasing free cytokinin in these cells and leading to shoot formation.

### EXAMPLE 12

### MODIFICATION OF THE POSITIVE SELECTION SYSTEM TO IMPROVE THE SELECTIVE EFFECT OF THE CYTOKININ GLUCURONIDES

It has already been shown (see Table 3, Example 3) that the naturally occurring β-glucuronidase in plants is inactive at relatively high pH values, i.e. at pH values of about 6 or more, while the introduced β-glucuronidase is active up to pH 8. By adding to the growth medium a compound which can raise the internal pH of the cells, e.g. ammonium nitrate, the selective shoot formation from the GUS-positive cells may be further improved, since it thereby becomes possible to block any background β-glucuronidase activity resulting from naturally occurring β-glucuronidase in the non-transformed cells.

The table below shows the number of shoots obtained from GUS-positive and GUS-negative tobacco leaf discs using various concentrations of BA3GN sodium salt and ammonium nitrate. The methods used were the same as those described in Example 3, with the exception that Gamborg B5 substrate was used instead of MSO substrate.

**TABLE 13**

| Effect of ammonium nitrate on selective shoot formation from BA3GN sodium salt treated leaf discs (pH = 7) | | | | | | | | | |
|---|---|---|---|---|---|---|---|---|---|
| Shoot formation (number of shoots and selectivity factor*) in GUS+ and GUS-leaf discs | | | | | | | | | |
| 7.5 mg/l BA3GN** | | | | 15 mg/l BA3GN** | | | 30mg/l BA3GN** | | |
| Ammonium nitrate conc.(mM) | GUS+ | GUS- | sel. * factor | GUS+ | GUS- | sel. * factor | GUS+ | GUS- | sel. * factor |
| 25 | 4 | 0 | >4x | 33 | 2 | 17x | 73 | 36 | 2x |
| 35 | 6 | 1 | 6x | 50 | 7 | 7x | 73 | 39 | 2x |
| 45 | 0 | 0 | - | 20 | 6 | 3x | 57 | 27 | 2x |
| 55 | 14 | 0 | >14x | 34 | 0 | >34x | 37 | 11 | 3x |
| 65 | 2 | 0 | >2x | 8 | 2 | 4x | 25 | 45 | 1x |
| Average | 26 | 1 | 26x | 145 | 17 | 9x | 265 | 158 | 2x |

| | | | | | | | | | |
|---|---|---|---|---|---|---|---|---|---|
| * The selectivity factor is the number of GUS-positive shoots divided by the number of GUS-negative shoots, and thus gives an indication of the selectivity of a given treatment ** sodium salt | | | | | | | | | |

It may be seen that the use of both BA3GN sodium salt and ammonium nitrate in appropriate concentrations leads to selective shoot formation in the GUS-positive leaf discs. For example, a combination of 15 mg/l BA3GN sodium salt and 55 mM ammonium nitrate gave 34 shoots from the GUS-positive leaf discs, while no shoots were formed on corresponding GUS-negative leaf discs subjected to the same treatment.

Another possibility for improving the selectivity of the positive selection system using cytokinin glucuronides is to add to the growth medium a substrate which after cleavage by β-glucuronidase results in a pH increase, thereby inhibiting the hydrolysis of cytokinin glucuronides in the non-transformed cells without inhibiting the effect of free cytokinin.

This principle was illustrated in an experiment on the effect of various concentrations of o-coumaryl-β-D-glucopyranuronic (CouGN) acid on shoot formation from GUS negative tobacco leaf discs induced by BA3GN sodium salt or BA. When o-coumaryl-β-D-glucopyranuronic acid is cleaved by the action of β-glucuronidase, o-coumaric acid is released. As mentioned above (see Example 1I), o-coumaric acid is spontaneously converted to coumarin. This involves the elimination of an acid group (see below) and thereby an increase of pH to a level at which the activity of the native plant β-glucuronidase is presumed to be reduced.

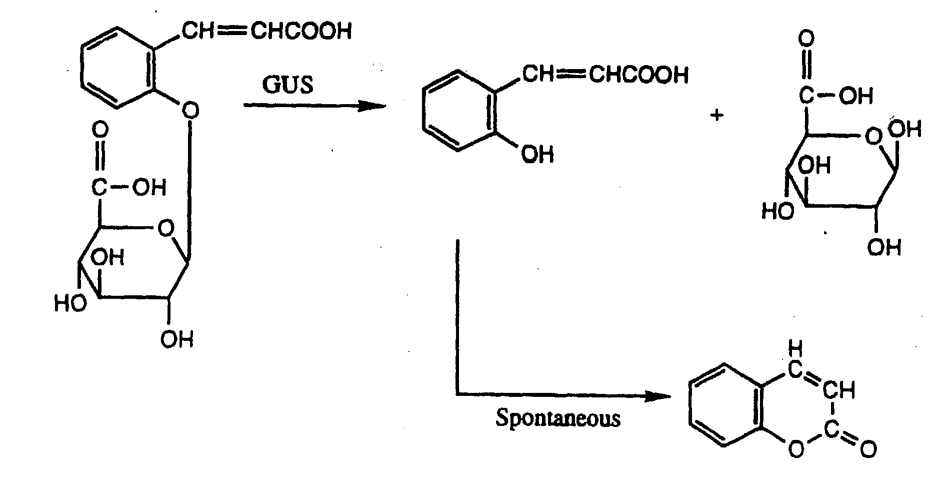

The experiment was performed using a BA concentration of 0.5 mg/l and a BA3GN sodium salt concentration of 10.0 mg/l. There were 12 discs per treatment, and the number of shoots was registered after 19 days. The results are shown below.

**TABLE 14**

| Effect of o-coumaryl-β-D-glucopyranuronic acid (CouGN) on shoot formation induced by BA or BA3GN sodium salt | | | | | |
|---|---|---|---|---|---|
| Conc. (mM) CouGN | Number of regenerated shoots | | | | Relative % BA/BA3GN* |
| | BA | relative % | BA3GN* | relative % | |
| 0 | 71 | 100 | 77 | 100 | 1.0 |
| 0.0625 | 66 | 93 | 78 | 101 | 0.9 |
| 0.125 | 71 | 100 | 63 | 82 | 1.2 |
| 0.25 | 56 | 79 | 79 | 103 | 0.8 |
| 0.5 | 71 | 100 | 56 | 73 | 1.4 |
| 1.0 | 70 | 99 | 4 | 5 | 19.8 |
| 2.0 | 65 | 92 | 6 | 8 | 11.5 |
| 3.0 | 37 | 49 | 0 | 0 | >49 |
| 4.0 | 36 | 47 | 0 | 0 | >47 |
| 5.0 | 16 | 21 | 0 | 0 | >21 |
| 6.0 | 12 | 16 | 0 | 0 | >16 |
| 7.0 | 13 | 17 | 0 | 0 | >17 |

| | | | | | |
|---|---|---|---|---|---|
| * sodium salt | | | | | |

The above table shows that the presence of o-coumaryl-β-D-glucopyranuronic acid in the growth medium inhibits shoot regeneration induced by BA3GN sodium salt but not by BA. Best results were obtained in this experiment using an o-coumaryl-β-D-glucopyranuronic acid concentration of about 3-4 mM. Several mechanisms could be involved in the reduction of shoot formation induced by BA3GN, including the following: 1) an increased pH due to the release of o-coumaric acid, as explained above, 2) substrate competition between CouGN and BA3GN, leading to a lower frequency of hydrolysis of BA3GN, and 3) a reduced transport of BA3GN into the cells. While the exact mechanisms involved in the observed reduction of shoot formation in the presence of CouGN were not determined, it is believed that an increased pH is likely to have been at least partially responsible. In any event, this experiment indicates that the selectivity of the positive selection system may be improved by using the introduced β-glucuronidase gene to establish a self-regulating mechanism which can significantly reduce the effect of any background enzyme.

### EXAMPLE 13

### PREPARATION OF GENETICALLY TRANSFORMED PLANTS

The following gives a general method which may be used for the preparation of genetically transformed plants.

### Plant material

Leaves (Nicotiana tabacum 'Wisconsin 38') are obtained from plants grown in vitro or in vivo. In the latter case, the leaves are sterilized prior to transformation. Sterilization may be performed by placing the leaves for 20 min. in a solution of 5% calcium hypochlorite containing 0.1 ml Tween 80 per 1 followed by washing 5 times in sterile water. In vitro plants are grown in containers on 1/2 MSO. (1/2 MSO is the same substrate as MSO in a 50% concentration except for agar, sugar and vitamins.)

The leaves are placed one at a time in a 14 cm Petri dish. They are then punched or cut into pieces of about 1 cm² without a major vein, the edges of the pieces consisting of tissue which has been cut. Any cut tissue which has been bleached by hypochlorite sterilization is removed.

### Cultivation of bacteria

One day before transformation a culture of bacteria is started by adding 2-3 ml of bacteria to 200 ml of LB medium in an Erlenmeyer flask. The bacteria is grown at 28°C with agitation (300 rpm).

### Transformation

The bacteria culture is diluted 50x or to OD 0.1 (at 660 nm) with 1/2 MSO immediately before transformation. Approximately 10 ml of the diluted bacteria suspension is poured into a 9 cm Petri dish, and the leaf pieces are dipped in this suspension for about 15 min. The leaf pieces are then removed and excess bacteria suspension is removed using sterile filter paper.

### Co-cultivation

The day before transformation a piece of sterile filter paper is placed on co-cultivation dishes (typically containing MSO substrate) and the leaf pieces which have been dipped in the bacteria suspension are placed upside down on the filter paper. The leaf pieces are incubated in a growth chamber with a cycle of 12 hours of light and 12 hours of darkness for 2 days.

### Selection/regeneration

The leaf pieces are transferred to Petri dishes containing cytokinin glucuronides as indicated and either 350 mg/l carbenicillin + 350 mg/l cefotoxime or 800 mg/l carbenicillin alone, in certain cases in combination with kanamycin sulfate. The leaf pieces are sub-cultivated after 3 weeks to the same medium, but without cytokinin glucuronides.

### Assay

Regenerated shoots are transferred to containers with 1/2 MSO. After about 2 weeks the X-gluc assay is performed on the green shoots. The shoots are sub-cultivated as necessary.

### Planting out

Genetically transformed shoots which have formed roots (and which are GUS-positive) are planted out in a growth chamber. The shoots are planted in a suitable growth medium, e.g. sphagnum. They are then covered with plastic bags and are grown for about 1 week, after which the two corners of the plastic bags are cut off. After another week the plastic bags are removed.

### EXAMPLE 14

### INDUCTION OF SHOOT FORMATION FROM PLANT TISSUE WITH AND WITHOUT AN INTRODUCED β-GLUCURONIDASE GENE USING STEROLS AND A DI-β-D-GLUCURONIDE

Tests similar to those described in Examples 5 and 6 were performed on GUS-positive and GUS-negative tobacco leaf discs using substrates containing 100 mg/l β-sitosterol, 100 mg/l cholesterol, 10 mg/l campesterol, 1.88 mg/l BA3GN sodium salt and various concentrations of the di-β-D-glucuronide glycyrrhizic acid in the form of a diammonium salt. In addition, half of the substrates contained 0.1 mM tridemorph. The number of shoots was registered after 17 days.

The results are shown in the following table.

**TABLE 15**

| Regenerated shoots per leaf disc | | | | |
|---|---|---|---|---|
| Glycyrrhizic acid* | Without tridemorph | | With tridemorph (0.1 mM) | |
| Conc. (mM) | GUS+ | GUS- | GUS+ | GUS- |
| 0.00125 | 1.6 | 0 | 0.4 | 0 |
| 0.0125 | 1.1 | 0 | 2.3 | 0 |
| 0.125 | 1.3 | 0.1 | 7.7 | 0.4 |
| 1.25 | 0 | 0 | 0 | 0 |
| 6.25 | 0 | 0 | 0 | 0 |

| | | | | |
|---|---|---|---|---|
| * diammonium salt | | | | |

It may be seen that the combination of sterols and the diammonium salt of glycyrrhizic acid leads to selective shoot formation in leaf discs containing an introduced β-glucuronidase gene. (As mentioned above (see Example 5) tridemorph inhibits the synthesis of sterols and thus has an inhibiting effect on shoot regeneration when the plant tissue is not supplied with sterols). While the selective shoot induction effect is seen using substrates both without and with tridemorph, the greatest number of shoots is obtained in the substrates containing tridemorph, and in particular with a glycyrrhizic acid diammonium salt concentration of 0.125 mM.

### EXAMPLE 15

### SELECTIVE INHIBITION OF BA3GN INDUCED SHOOT FORMATION FROM WILD TYPE (GUS-) TOBACCO LEAF DISCS

Experiments were performed as described in Example 3 using the tobacco variety'Burley' instead of 'Wisconsin 38'. Methyl-β-D-glucuronide (MG), which is hydrolysed to methanol and glucuronic acid by GUS, was added to the substrate in various concentrations, along with either 1 mg/l BA or 15 mg/l BA3GN sodium salt. Methanol has been shown to inhibit the native GUS enzyme without affecting the introduced E. coli enzyme very much (Kosugi et al., 1990, Plant Sci., 133-120), while glucuronic acid liberated from methyl-β-D-glucuronide is a product inhibitor of GUS enzymes. By adding MG instead of the two compounds independently, the hydrolysis products are produced locally and concentrated in the compartment where the enzyme is localized. The results obtained are shown in Table 16 below.

**TABLE 16**

| Compound | Conc. | Shoots | per leaf disc | Ratio |
|---|---|---|---|---|
| | mg/l | BA | BA3GN | BA/BA3GN |
| Methyl-β-glucuronide | 0 | 1.00 | 0.50 | 2.0 |
| | 1000 | 1.91 | 1.00 | 1.9 |
| | 3300 | 2.25 | 0 | - |
| | | | | |
| Glucuronic acid | 0 | 1.00 | 0.50 | 2.0 |
| | 1000 | 0.33 | 0.08 | 4.1 |
| | 3300 | 0.83 | 0 | - |
| | 10,000 | 0.42 | 0 | - |
| BA 1 mg/l | | | | |
| BA3GN 15 mg/l | | | | |

The above results show that the addition of either MG or glucuronic acid leads to a reduction of the number of shoots obtained on the BA3GN substrate compared to the number of shoots obtained on the BA substrate. By treating tissue with MG before expression of the introduced GUS enzyme, it may therefore be possible to selectively eliminate or reduce the activity or the effect of the native GUS enzyme during the selection process. Differences between the introduced and the native GUS enzymes with regard to inhibition due to substrate competition, sensitivity to substrate inhibition, amount of enzyme (activity), and sensitivity to product inhibition may also account for the effects of MG and other compounds having a similar effect on wild type tissues treated with glucuronides.

It is also possible that MG functions by competing with the uptake of other glucuronides such as BA3GN. If this is the case, MG could be used to reduce or eliminate uptake of other glucuronides in wild type cells. Introduction of a gene encoding a GUS enzyme that is secreted or a gene encoding a glucuronide permease might be used to select transgenic cells if uptake is inhibited by e.g. addition of MG to the substrate. In the case of glucuronide permease, only transgenic cells would take up the glucuronide (e.g. BA3GN) and due to the general occurrence of the native enzyme in plants (see e.g. Example 3), the compound would be activated inside the cells expressing the permease (or another protein which facilitates the uptake of glucuronides).

It is also interesting that glucuronic acid itself is able to selectively inhibit the effect of BA3GN, presumably by blocking the effect of BA released through cleavage of BA3GN by GUS.

### EXAMPLE 16

### USE OF POSITIVE SELECTION AND A COMBINATION OF POSITIVE AND NEGATIVE SELECTION TO IMPROVE THE EFFICIENCY OF SELECTION OF TRANSGENIC CELLS, TISSUES OR SHOOTS FROM RECALCITRANT SPECIES

Sugar beet is a very recalcitrant species with regard to producing transgenic plants. Many untransformed shoots are "selected" under conditions which give rise to transgenic shoots in ordinary transformation systems. The same was found to be the case when positive selection experiments (without addition of kanamycin) were performed using 5-15 mg/l BA3GN sodium salt under the conditions described in the following.

In Example 11 (see Table 12), the combination of positive and negative selection was found to reduce the number of "selected" non-transformed shoots. Therefore, the combination of positive and negative selection was tested on sugar beet, and this was found to give advantageous results.

Transformation was carried out using cotyledon explants as described below.

Seeds were germinated for 4 days in darkness on a substrate containing 0.7 g/l of agarose and 2 g/l of sucrose. The seedlings were then transferred to a Nunc container containing 1/2 x MSO substrate and cultured for 3 days in the light. The cotyledons were removed from the seedlings, and the cotyledon explants were then brushed on the petiole with a small brush containing an Agrobacterium suspension, the Agrobacterium containing 35S-NPTII and 35S-GUS (OD 660=0.1). The cotelydons were then co-cultivated for 4 days on a substrate containing 1/10 MSO substrate and 200 µM acetosyringone. The transformed explants were transferred to an MSO substrate supplemented with 0.25 mg/l of BA or 15 mg/l of BA3GN sodium salt (instead of BAP), 0.025 mg/l of naphthyl acetic acid, 400 mg/l of kanamycin, 800 mg/l of carbenicillin and 25 mg/ml of vancomycin, and the explants were incubated for 21 days on this substrate. The regenerated shoots were then transferred to containers containing the same substrate. After 52 days on this substrate all the shoots were transferred to MSO substrate supplemented with 800 mg/l carbenicillin, 25 mg/l vancomycin and 0.1 mg/l BA. After 14 days GUS assays as described in Example 3 were performed on the selected plant material.

The results are shown in the table below.

**TABLE 17**

| Transformation of sugar beet Combination of positive and negative selection | | | |
|---|---|---|---|
| | No. of explants | GUS+ shoots | GUS+ shoots (%) |
| Negative selection | 100 | 0 | 0% |
| Positive and negative selection | 177 | 4 | 2.3% |
| Negative selection: | 400 mg/l kanamycin sulphate + 1 mg/l BAP | | |
| Positive and negative selection: | 400 mg/l kanamycin sulphate + 15 mg/l BA3GN | | |

It can be seen that with the combination of positive and negative selection, transgenic shoots are produced under conditions in which no transgenic shoots are produced using the traditional negative selection system. This shows that the use of the positive selection system is very advantageous compared to the use of pure negative selection systems in sugar beet.

This in turn indicates that the use of positive selection (alone in combination with negative selection) may make it possible to produce transgenic plants in other recalcitrant species in which only low transformation/selection frequencies are obtained or in which no transgenic plants are able to be selected at all using negative selection systems.

### EXAMPLE 17

### POSITIVE SELECTION SYSTEMS BASED ON THE USE OF INACTIVE N-SOURCES MADE AVAILABLE BY THE INTRODUCTION OF METABOLISING GENES

Experiments were performed as described in Example 3, but the normal nitrogen content of the MSO substrate was reduced to zero. Instead, the nitrogen compounds indicated in Table 18 were added. The substrate contained 1 mg/l BA. Substrates containing ammonium nitrate were used as positive controls.

These experiments were performed to investigate whether opines are inactive or whether they can be used by plant cells as nitrogen sources in substrates not containing any other nitrogen source. Because genes encoding enzymes which metabolise opines are well known, the major prerequisite for using opines in a positive selection system is the identification of opines that cannot be used as a nitrogen source for plant tissues and cells not containing introduced genes which enable the plant cells to utilize the opines in question. The results are given below.

**TABLE 18**

| The effect of opines on shoot formation from tobacco leaf discs on substrates without nitrogen (number of shoots per 9 leaf discs) | | | | | |
|---|---|---|---|---|---|
| | Concentration (mM) | | | | |
| Compound | 0 | 10 | 20 | 40 | 80 |
| Octopine | 13 | 11 | 15 | 15 | 6 |
| Mannopine | 13 | >50 | >50 | >50 | >50 |
| Nopaline | 13 | 18 | 19 | 11 | 8 |
| Ammonium nitrate (control) | 13 | >50 | >50 | >50 | >50 |

In tested substrates containing no nitrogen, a few shoots were regenerated. This may have been possible because nitrogen can be mobilised from the tissue in the explant. To avoid any shoot formation in the treatments without any nitrogen, the explants can be starved for nitrogen by growing the parent cultures on nitrogen-free substrates before use or by pre-treating the explants on a nitrogen free substrate, e.g. without shoot inducing hormones.

It was surprisingly found that octopine and nopaline cannot support shoot formation, while mannopine can function as a good nitrogen source and support shoot formation from the leaf discs.

It is likely that the reason why nopaline and octopine cannot function as nitrogen sources is that these compounds are not taken up, metabolised or hydrolysed into usable compounds. It is well known that organisms containing genes involved in the transport and metabolism of opines, e.g. Agrobacterium, are able to use opines as a nitrogen source, while Agrobacterium or other bacteria strains not containing genes encoding opine metabolism are not able to grow on substrates containing only opines as a nitrogen source.

Based on these results, positive selection systems may be established by introducing one or more opine metabolism or transport genes into transgenic plant cells using selection substrates not containing or with a reduced level of nitrogen sources other than e.g. nopaline or octopine. The identification or isolation of genes or genetic material conferring to the recipient the capacity to utilise octopine and nopaline has been described in the literature: see e.g. C. Beaulieu et al., 1988, Can. J. Microbiol., 38: 843-49; P. M. Klapwijk et al., 1976, J. Gen. Microbiol., 96: 155-163; C. L. Schardl and C. I. Kado, 1983, Mol. Gen. Genet., 191: 10-16 or H. Wabiko et al., 1990, J. Gen. Microbiol. 136: 97-103. Upon isolation of genetic material encoding opine metabolism, eukaryotic organisms may be transformed according to standard procedures described in the literature with appropriate sequences necessary for the functioning of the genes for opine metabolism.

Based on the above results, it is likely that other opines and corresponding catabolizing genes can be used in a similar manner, and it further appears likely that other inactive N-sources identified by similar means and their corresponding genes can similarly be used, e.g. amides in combination with amidases, peptides in combination with specific peptidases, etc.

### EXAMPLE 18

### PRODUCTION OF TRANSGENIC CALLUS FROM RECALCITRANT SPECIES USING POSITIVE SELECTION IN COMBINATION WITH NEGATIVE SELECTION

Experiments were performed as described in Example 11, although with certain modifications. The plant species used were the very recalcitrant breeding lines "V486" of winter oil seed rape and "S487" of summer oilseed rape. Seeds were sterilised and germinated as described in Example 16. Hypocotyls were used as explants and were inoculated and co-cultured as described in Example 11. After co-cultivation the explants were transferred to MSO substrate containing 0.1 mg/l naphthylacetic acid, 0.01 mg/l gibberellic acid (GA3), 500 mg/l carbenicillin, 50 mg/l kanamycin sulphate and 6.0 g/l agarose. The substrate contained in addition either 1 mg/l BA or 3.75, 7.5 or 15.0 mg/l BA3GN sodium salt. The pH was adjusted to 5.8. The Agrobacterium used contained in its T-DNA a GUS gene and a neomycinphosphotransferase II gene driven by 35S promoters. GUS assays were performed after 8 weeks and callus showing an intense blue staining in most of the callus cells was registered as being GUS+.

**TABLE 19**

| Production of transgenic callus from oilseed rape using positive selection in combination with negative selection | | | | |
|---|---|---|---|---|
| Oilseed rape, winter type "V486" | | | | |
| | BA3GN (mg/l) | | | BA (mg/l) |
| | 3.75 | 7.5 | 15.0 | 1 |
| Explants with callus | 19.0% | 23.0% | 30.6% | 0% |
| GUS+ callus per explant | 1.4% | 1.4% | 2.3% | 0% |
| GUS+ callus per callus | 7.1% | 5.9% | 7.5% | 0% |

| Oilseed rape, summer type "S487" | | | | |
|---|---|---|---|---|
| | BA3GN (mg/l) | | | BA (mg/l) |
| | 3.75 | 7.5 | 15.0 | 1 |
| Explants with callus | 5.2% | 9.2% | 6.3% | 0% |
| GUS+ callus per explant | 1.7% | 2.3% | 0.9% | 0% |
| GUS+ callus per callus | 33.3% | 25.0% | 14.2% | 0% |

These experiments show that with these very recalcitrant types of oilseed rape, only positive selection in combination with negative selection allowed selection of transgenic callus, while no GUS positive callus was obtained using traditional negative selection (substrates with BA instead of BA3GN sodium salt).

This shows that the introduction of the positive selection systems is very advantageous compared to the pure negative selection systems, also in callus systems. It also shows that the use of positive selection (alone or in combination with negative selection) may make it possible to produce transgenic plants in other recalcitrant species in which only low transformation/selection frequencies are obtained or in which no transgenic plants at all are selected using negative selection systems.

After transgenic callus has been selected, it can be regenerated into transgenic shoots, e.g. on a substrate containing a cytokinin in combination with a low concentration of auxins. No selection is needed during this process.

## Claims

1. A method of selecting genetically transformed plant cells from a population of cells, wherein the genetically transformed plant cells are transformed with a desired expressible nucleotide sequence containing a regulatory sequence enabling its expression in the transformed cells and a co-introduced expressible nucleotide sequence also containing a regulatory sequence enabling its expression in the transformed cells, which method comprises supplying the said population with a compound which can be metabolized by the expression product of the co-introduced expressible nucleotide sequence which has been introduced with the desired expressible nucleotide sequence into the said transformed cells, so as to provide the transformed cells with a physiological advantage when compared to the non-transformed cells, wherein the compound is not an antibiotic or herbicide and has no direct adverse effect on the non-transformed cells.

2. A method according to claim 1. In which the compound is selected from the group consisting of cytokinins, auxins, gibberellins, vitamins, carbohydrates, opines, proteins, sterols and saponins.

3. A method according to either of claims 1 or 2, wherein the said co-introduced expressible nucleotide sequence encodes β-glucuronidase when the compound is a cytokinin glucuronide,

4. A method according to the preceding claim, wherein native β-glucuronidase activity is reduced by supplying to the culture medium a pH regulating compound which provides the culture medium, the cells or compartments of the cells with a pH of between 5.5 and 8.5.

## Patentansprüche

1. Ein Verfahren zur Selektion genetisch transformierter Pflanzenzellen aus einer Zellpopulation, worin die genetisch transformierten Pflanzenzellen mit einer erwünschten exprimierbaren Nukleotidsequenz, die eine regulatorische Sequenz enthält, die ihre Expression in den transformierten Zellen ermöglicht, und einer mit-eingeführten exprimierbaren Nukleotidsequenz, die ebenfalls eine regulatorische Sequenz enthält, die ihre Expression in den transformierten Pflanzenzellen ermöglicht, transformiert sind, welches Verfahren umfasst, besagte Population mit einer Verbindung zu versehen, die durch das Expressionsprodukt der mit-eingeführten exprimierbaren Nukleotidsequenz, die mit der erwünschten exprimierbaren Nukleotidsequenz in besagte transformierte Zellen eingeführt wurde, metabolisiert werden kann, um so die transformierten Zellen verglichen mit den nicht transformierten Zellen mit einem physiologischen Vorteil zu versehen, wobei die Verbindung kein Antibiotikum oder Herbizid ist und keine direkte nachteilige Wirkung auf die nicht transformierten Zellen hat.

2. Verfahren gemäß Anspruch 1, in dem die Verbindung ausgewählt ist aus der Gruppe, die aus Cytokinen, Auxinen, Gibberellinen, Vitaminen, Kohlenhydraten, Opinen, Proteinen, Sterolen und Saponinen besteht.

3. Verfahren gemäß einem der Ansprüche 1 oder 2, worin besagte mit-eingeführte exprimierbare Nukleotidsequenz β-Glucuronidase codiert, wenn die Verbindung ein Cytokinglucuronid ist.

4. Verfahren gemäß dem vorhergehenden Anspruch, worin die ursprüngliche β-Glucuronidaseaktivität reduziert wird, indem man das Kulturmedium mit einer pH-regulierenden Verbindung versieht, die das Kulturmedium, die Zellen oder Kompartimente der Zellen mit einem pH zwischen 5,5 und 8,5 versieht.

## Revendications

1. Méthode de sélection de cellules végétales transformées génétiquement à partir d'une population de cellules, où les cellules végétales transformées génétiquement sont transformées avec une séquence nucléotidique exprimable souhaitée, contenant une séquence régulatrice permettant son expression dans les cellules transformées, et une séquence nucléotidique exprimable co-introduite, contenant aussi une séquence régulatrice permettant son expression dans les cellules transformées, laquelle méthode comprenant la fourniture, à ladite population, d'un composé qui peut être métabolisé par le produit d'expression de la séquence nucléotidique exprimable co-introduite, qui a été introduite avec la séquence nucléotidique exprimable souhaitée dans lesdites cellules transformées, pour conférer aux cellules transformées un avantage physiologique comparé aux cellules non transformées, où le composé n'est pas un antibiotique ou un herbicide et n'a pas d'effet défavorable direct sur les cellules non transformées.

2. Méthode selon la revendication 1, dans laquelle le composé est choisi parmi le groupe constitué par les cytokinines, les auxines, les gibbérélines, les vitamines, les glucines, les opines, les protéines, les stérols et les saponines.

3. Méthode selon la revendication 1 ou 2, dans laquelle ladite séquence nucléotidique exprimable co-introduite code pour la β-glucuronidase lorsque le composé est un cytokinine-glucuronide.

4. Méthode selon la revendication précédente, dans laquelle l'activité de la β-glucuronidase native est réduite grâce à la fourniture, au milieu de culture, d'un composé régulant le pH qui confère au milieu de culture, aux cellules ou aux compartiments des cellules un pH compris entre 5,5 et 8,5.
